(19) Europäisches Patentamt
European Patent Office
Office européen des brevets

(11) **EP 3 747 908 A1**

(12) **DEMANDE DE BREVET EUROPEEN**

(43) Date de publication:
**09.12.2020 Bulletin 2020/50**

(51) Int Cl.:
**C07K 16/28** *(2006.01)*   **C07K 16/30** *(2006.01)*
**A61K 39/395** *(2006.01)*   **A61P 35/00** *(2006.01)*

(21) Numéro de dépôt: **20184087.3**

(22) Date de dépôt: **05.06.2015**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.06.2014 FR 1455177**

(62) Numéro(s) de document de la (des) demande(s) initiale(s) en application de l'article 76 CBE:
**15732846.9 / 3 152 234**

(71) Demandeurs:
- **CENTRE NATIONAL DE LA RECHERCHE SCIENTIFIQUE -CNRS-
  75016 Paris (FR)**
- **Institut Gustave Roussy
  94800 Villejuif (FR)**
- **Cellvax
  93230 Romainville (FR)**
- **Université de Lille
  59000 Lille (FR)**
- **Université Paris-Saclay
  91190 Saint-Aubin (FR)**

(72) Inventeurs:
- **DELHEM, Nadira
  59700 Marcq en Baroeul (FR)**
- **BUSSON, Pierre
  92160 Antony (FR)**
- **MORALES, Olivier
  59000 Lille (FR)**
- **BARJON, Clément
  94800 Villejuif (FR)**
- **MRIZAK, Dhafer
  59000 Lille (FR)**
- **LHUILLIER, Claire
  94270 Kremlin Bicetre (FR)**
- **MUSTAPHA, Rami
  59000 Lille (FR)**

(74) Mandataire: **Plasseraud IP
66, rue de la Chaussée d'Antin
75440 Paris Cedex 09 (FR)**

Remarques:
Cette demande a été déposée le 03.07.2020 comme demande divisionnaire de la demande mentionnée sous le code INID 62.

(54) **ANTICORPS DIRIGÉ CONTRE LA GALECTINE 9 ET INHIBITEUR DE L'ACTIVITÉ SUPPRESSIVE DES LYMPHOCYTES T RÉGULATEURS**

(57) L'invention concerne un anticorps dirigé contre la galectine 9 et inhibiteur de l'activité suppressive des lymphocytes T régulateurs, ainsi que l'utilisation de cet anticorps pour le traitement de maladies associées à l'activité suppressive des lymphocytes T régulateurs, notamment le traitement de cancer.

EP 3 747 908 A1

**Description**

L'invention concerne un anticorps dirigé contre la galectine 9 et inhibiteur de l'activité suppressive des lymphocytes T régulateurs, ainsi que l'utilisation de cet anticorps pour le traitement de maladies associées à l'activité suppressive des lymphocytes T régulateurs.

Les lymphocytes T humains se caractérisent par l'expression d'un marqueur membranaire appelé CD3 et d'un récepteur spécifique, le TCR (T cell receptor), qui est directement impliqué dans la reconnaissance spécifique d'un antigène. Cette reconnaissance antigénique par le lymphocyte T naïf induit l'activation de la réponse immunitaire primaire se traduisant par une modification du phénotype et de l'activité des lymphocytes T.

Différents types de populations lymphocytaires T vont se développer, par exemple les cellules "effectrices" ou lymphocytes T effecteurs, qui vont assurer des fonctions spécialisées pour défendre l'organisme. Ainsi, les lymphocytes T CD4+, encore appelés lymphocytes T auxiliaires sécrètent des cytokines majeures aidant notamment les lymphocytes B dans leur fonction humorale (production d'anticorps spécifiques) et les lymphocytes T CD8+ dans leur activité cytotoxique.

Une autre population de lymphocytes T CD4+ est constituée par les lymphocytes T régulateurs naturels, ci-après appelés plus brièvement « lymphocytes T régulateurs ». Ils sur-expriment constitutivement la molécule CD25 (les lymphocytes T régulateurs peuvent ainsi également être appelés « CD4+CD25+ ») et le facteur de transcription Foxp3. Ce faible pourcentage de lymphocytes T CD4+CD25+ a la particularité de réguler négativement les acteurs de la réponse immune qui auraient reconnu divers auto-antigènes par leurs TCR. Les lymphocytes T régulateurs jouent ainsi un rôle majeur dans la physiologie du système immunitaire en particulier pour protéger l'organisme contre l'émergence de maladies auto-immunes.

Cependant, il a été mis en avant qu'en situation pathologique, les lymphocytes T régulateurs peuvent induire une immunosuppression inappropriée, qui favorise alors la croissance tumorale ou la persistance de pathogènes infectieux (virus, bactéries, parasites...). De nombreuses études ont ainsi montré que les lymphocytes T régulateurs diminuent les réponses immunitaires anti-tumorales ou anti-virales, notamment en inhibant de manière inappropriée l'activité des lymphocytes T effecteurs, favorisant ainsi la persistance des virus et la progression tumorale dans une grande majorité des cancers.

Les mécanismes par lesquels les lymphocytes T régulateurs exercent leurs effets suppresseurs sur les lymphocytes T effecteurs sont encore mal connus. Cependant, diverses études ont mis en évidence différents mécanismes par lesquels les lymphocytes T régulateurs pourraient supprimer la réponse immunitaire. Parmi les explications possibles, des études ont par exemple mis en évidence que les lymphocytes T régulateurs Foxp3+ peuvent lyser les lymphocytes T effecteurs via la production de granzymes/perforines (1) ou en déprivant en IL-2 les lymphocytes t effecteurs ou encore en inhibant la prolifération des lymphocytes T effecteurs, notamment en exprimant des molécules de surface comme la galectine 1 qui interagit avec des récepteurs exprimés sur les lymphocytes T effecteurs et induit l'arrêt du cycle cellulaire des lymphocytes T effecteurs (2).

Le rôle physiopathologique des Treg dans les cancers, a ainsi encouragé l'émergence d'une nouvelle stratégie thérapeutique anti-tumorale. Elle consiste à neutraliser les facteurs inhibiteurs de la réponse immunitaire, et notamment les lymphocytes T régulateurs, ou en d'autres termes à briser la tolérance vis-à-vis des antigènes tumoraux.

En effet, dans le but d'inverser la balance entre lymphocytes T régulateurs et lymphocytes T effecteurs dans le contrôle de l'immunité anti-tumorale, de nombreuses équipes ont cherché à développer des stratégies thérapeutiques visant à inhiber les lymphocytes T régulateurs CD4+CD25+, notamment en ciblant, avec des anticorps monoclonaux, des molécules de surface exprimées par ces lymphocytes T régulateurs, et notamment celles intervenant dans l'activité suppressive de ces lymphocytes T régulateurs.

Des analyses réalisées chez le rongeur ont par exemple montré que l'inhibition des lymphocytes T régulateurs CD4+CD25+ par un anticorps monoclonal dirigé contre le récepteur alpha de l'IL-2 (CD25) favorisait l'activation et l'expansion de cellules T effectrices inhibant ainsi la croissance tumorale (3). Cependant, CD25 est également exprimé par les cellules T effectrices activées. Ainsi cette stratégie est à prendre avec précaution dans la mesure où elle peut également favoriser l'élimination des lymphocytes T effecteurs.

Il a également été documenté que l'activation de la signalisation par le GITR via un anticorps anti-GITR était capable d'inhiber l'activité suppressive des lymphocytes T régulateurs (4). Dans ce contexte, l'utilisation d'un anticorps anti-GITR dans le traitement de tumeurs murines a permis d'augmenter la réponse anti-tumorale des lymphocytes T CD4+ et CD8+, et ce de manière plus efficace lorsque la tumeur est déjà installée. Cependant, les lymphocytes T effecteurs activés expriment eux aussi le GITR. Par conséquent, il existe également un risque de suppression de lymphocytes T effecteurs lors de l'utilisation d'un anticorps anti-GITR.

La stratégie visant à inhiber les lymphocytes T régulateurs a également été envisagée par l'utilisation d'un anticorps anti-CTLA4, marqueur exprimé par les lymphocytes T régulateurs. Ainsi, dans un modèle de souris présentant un KO (KnockOut) du CTLA-4 dans les lymphocytes T régulateurs, ou lors de l'utilisation d'anticorps anti-CTLA-4, il a été montré une augmentation de l'activité des lymphocytes T effecteurs et une diminution de la suppression médiée par les lymphocytes T régulateurs, conduisant à une inhibition de la croissance tumorale (5). Cependant, les lymphocytes T effecteurs activés expriment également le marqueur CTLA-4. Par conséquent, il existe, là encore, un risque de suppression des lymphocytes T effecteurs par l'utilisation d'un anticorps anti-GITR.

Ainsi, malgré l'effet prometteur de ces différentes molécules, un des obstacles à la déplétion spécifique des lymphocytes T régulateurs est le manque de spécificité de leurs marqueurs de surface. En effet, les protéines de surface CD25, CTLA-4 ou GITR, exprimées par les lymphocytes T régulateurs sont également des marqueurs d'activation des lymphocytes T effecteurs. L'utilisation de ces protéines comme cibles pour la déplétion des lymphocytes T régulateurs a ainsi pour effet indésirable l'élimination de nombreux lymphocytes T effecteurs CD4+ et CD8+, indispensables à la régression tumorale. Dans ce contexte, il reste très difficile de cibler spécifiquement les lymphocytes T régulateurs dans des protocoles thérapeutiques.

Par conséquent, il existe toujours un besoin de composés permettant d'inhiber sélectivement et efficacement l'activité suppressive des lymphocytes T régulateurs.

Un des buts de l'invention est ainsi de fournir un anticorps qui soit dirigé contre un marqueur spécifique des lymphocytes T régulateurs et qui permette l'inhibition de l'activité suppressive des lymphocytes T régulateurs, sans altérer la fonction des lymphocytes T effecteurs.

Les inventeurs ont le mérite d'avoir mis en évidence qu'une molécule, la galectine 9, est exprimée de manière spécifique par les lymphocytes T régulateurs lors de l'activation et qu'un anticorps dirigé contre cette molécule permet d'inhiber l'activité suppressive des lymphocytes T régulateurs, et ce de manière spécifique, c'est-à-dire sans inhiber de lymphocytes T effecteurs. Par ailleurs, il a également été démontré qu'un tel anticorps est capable de neutraliser la conversion des lymphocytes T CD4+ conventionnels en T CD4+ immunosuppresseurs induite par la galectine 9, favorisant ainsi le maintien d'une réponse immunitaire anti-tumorale chez le patient soigné selon l'invention.

Il a ainsi été découvert qu'un tel anticorps dirigé contre la galectine 9 et inhibiteur de l'activité suppressive des lymphocytes T régulateurs pouvait être utilisé dans le traitement de maladies associées à l'activité suppressive des lymphocytes T régulateurs.

On entend ici par « *maladie associée à l'activité suppressive des lymphocytes T régulateurs* » toute maladie (non auto-immune) dans laquelle l'activité suppressive des lymphocytes T régulateurs joue un rôle, notamment en favorisant le développement ou la persistance de la maladie. En particulier, il a été démontré que l'activité suppressive des lymphocytes T régulateurs favorise le développement de tumeurs. L'invention vise donc plus particulièrement les cancers dans lesquels l'activité suppressive des lymphocytes T joue un rôle.

La galectine 9, pouvant être appelée de manière plus concise « gal9 », fait partie de la famille des galectines. Les galectines, ou lectines de type S, constituent une famille composée de quinze membres chez les vertébrés, dont dix chez l'homme. La galectine 9 interagit préférentiellement avec les résidus béta-galactosides des glycoprotéines et des glycolipides. Chez l'homme, la galectine 9 existe sous trois isoformes, longue, moyenne et courte.

Plusieurs études ont été effectuées afin de déterminer le lien existant entre le développement de cancers et les galectines, notamment la galectine 9. Cependant, il est considéré dans la plupart de ces études que la galectine 9 a une activité cytotoxique contre les lymphocytes T activés (que ce soit CD4+ ou CD8+) et n'a pas d'activité cytotoxique contre les lymphocytes T non activés.

Par exemple, la demande de brevet EP1586325 part du postulat que, *in vitro,* la galectine 9 induit l'apoptose des cellules tumorales, notamment dans des cellules malignes ou métastasiques, mais pas des cellules normales. La demande EP1586325 vise ainsi un médicament comprenant de la galectine 9 ou des molécules induisant la production et/ou la libération de galectine 9, etc.

L'objectif était donc d'utiliser la galectine 9 ou des facteurs permettant d'augmenter la galectine 9, tandis que la présente invention tend, au contraire, à l'inhiber.

La présente invention a ainsi pour objet un anticorps dirigé contre la galectine 9 et inhibiteur de l'activité suppressive des lymphocytes T régulateurs.

En d'autres termes, l'invention concerne un anticorps dirigé contre la galectine 9, caractérisé en ce qu'il est inhibiteur de l'activité suppressive des lymphocytes T régulateurs.

En effet, l'invention repose sur les constatations inattendues faites par les inventeurs, qui ont observé (i) d'une part, que la galectine 9 est directement exprimée par les lymphocytes T régulateurs et que son expression est augmentée lors de leur activation et (ii) d'autre part que la galectine 9 est très faiblement exprimée par les lymphocytes T effecteurs et que cette expression disparait lors de l'activation. Par ailleurs, les inventeurs ont observé que l'inhibition de la galectine 9 par un anticorps permet l'inhibition de l'activité suppressive des lymphocytes T régulateurs.

Par « *lymphocytes T régulateurs* », on entend la sous-population de lymphocytes T régulateurs naturels, aussi appelée Treg, caractérisée par une expression constitutive du CD25, du CTLA-4, et du GITR et par une expression spécifique du facteur de transcription Foxp3. Les lymphocytes T régulateurs plus particulièrement visés par la présente invention sont donc les lymphocytes T régulateurs naturels, ou nTreg.

On entend par « *activité suppressive des lymphocytes T régulateurs* » l'activité immunosuppressive que les lymphocytes T régulateurs exercent sur les lymphocytes T effecteurs, une fois activés, en situation pathologique et qui favorise en particulier la croissance tumorale. De préférence, l'activité suppressive des lymphocytes T régulateurs peut être entendue comme l'activité diminuant les réponses immunitaires anti-tumorales par l'inhibition de l'activité des lymphocytes T effecteurs. L'activité suppressive des lymphocytes T régulateurs peut être analysée selon différentes techniques connues de l'homme du métier. Par exemple, une méthode MLR (Mixed Leukocyte Reaction) peut être effectuée, laquelle met en œuvre une co-culture de lymphocytes Treg et de cellules immunitaires (PBMC totaux ou T CD4+) autologues ou hétérologues. Celle-ci peut être réalisée par des tests de prolifération basée sur (i) l'incorporation de radioéléments tels que la Thymidine Tritiée ou (ii) l'incorporation d'EdU (5-éthynyl-2'-deoxyuridine) qui s'incorpore lors de la synthèse de l'ADN et qui suite à une réaction enzymatique permettra l'émission d'une fluorescence (Click-it EdU Proliferation test) ou (iii) cytométrie de flux (CSFE).

L'effet inhibiteur d'un anticorps selon l'invention peut ainsi par exemple être analysé par une telle méthode MLR, réalisée en présence de l'anticorps testé.

On peut ici se référer à la partie « Exemples », « Matériels et méthodes » pour plus de détails concernant la mise en œuvre d'une telle méthode d'analyse.

Les termes « *anticorps* » et « *immunoglobuline* » sont utilisés indifféremment et font référence à des molécules d'immunoglobuline ou des portions immunologiquement actives de molécules d'immunoglobuline, i.e. des molécules comprenant les sites de fixation spécifique d'un antigène donné. Le terme anticorps couvre non seulement les molécules anticorps entières mais aussi les fragments d'anticorps et les variants (incluant les dérivés tels que les anticorps humanisés) d'anticorps et de fragments d'anticorps.

Les immunoglobulines sont bien connues de l'homme du métier et sont constituées de deux chaînes lourdes reliées l'une à l'autre par des ponts disulfure, chaque chaîne lourde étant reliée à une chaîne légère par un pont disulfure. Il existe deux sortes de chaînes légères, les chaînes lambda ($\lambda$) et kappa (K). Il existe cinq classes principales de chaînes lourdes qui déterminent l'activité fonctionnelle de l'anticorps : IgM, IgD, IgG, IgA et IgE.

Chaque chaîne contient des domaines de séquences distincts. La chaîne légère comprend deux domaines, un domaine (ou région) variable (VL) et un domaine constant (CL). La chaîne lourde comprend quatre ou cinq domaines selon les classes d'anticorps, un domaine variable (VH) et trois, voire quatre, domaines constants (CH1, CH2, CH3 et éventuellement CH4). Les régions variables des chaînes légères (VL) et lourdes (VH) déterminent la spécificité pour l'antigène et le site de fixation sur cet antigène.

Les domaines constants des chaînes légères (CL) et lourdes (CH) confèrent à l'anticorps des propriétés biologiques importantes comme l'association des chaînes anticorps entre elles, la mobilité à travers le placenta, la fixation du complément et/ou la fixation aux récepteurs Fc (FcR). Le fragment Fv correspond à la partie V-terminale du fragment Fab, décrit ci-après, de l'immunoglobuline, et comprend les portions variables d'une chaîne légère et d'une chaîne lourde (VL et VH). La spécificité de l'anticorps réside dans la complémentarité structurelle entre le site de reconnaissance de l'anticorps et le déterminant antigénique. Le site de reconnaissance de l'anticorps est constitué essentiellement de résidus provenant de régions hypervariables ou déterminant la complémentarité (« Complementary Determining Régions » ou CDRs). Occasionnellement, les résidus provenant de régions non hypervariables ou des régions charpentes ou d'armature ("framework" ou FR) influencent la structure générale du domaine et donc les sites de reconnaissance. Le terme "régions de complémentarité" (CDR) se rapporte à des séquences d'acides aminés qui, ensemble, définissent l'affinité de la fixation et la spécificité de la région Fv naturelle du site de fixation d'une immunoglobuline native. Chacune des chaînes légères et chacune des chaînes lourdes d'une immunoglobuline possède trois régions CDRs désignées par L-CDR1, L-CDR2, L-CDR3 et H-CDR1, H-CDR2, H-CDR3, respectivement. Un site de liaison d'un antigène comprend par conséquence six CDRs. Les régions charpentes (FR) se rapportent aux séquences d'acides aminés interposées entre les CDRs, c'est-à-dire des portions des régions variables des chaînes légères et lourdes des immunoglobulines qui sont relativement conservées entre différentes immunoglobulines d'une même espèce.

Un anticorps selon l'invention peut être un anticorps monoclonal ou polyclonal. De préférence, un anticorps selon l'invention est un anticorps monoclonal.

Le terme *"anticorps monoclonal"* ou *"mAb"* (pour « monoclonal Antibody ») désigne un anticorps de composition en acides aminés unique, qui est dirigé contre un antigène spécifique et qui peut être produit par un seul clone de cellules B, ou hybridome. Les anticorps monoclonaux peuvent également être recombinants, c'est-à-dire être produits par des techniques d'ingénierie des protéines.

Le terme « *Fab* » désigne un fragment d'anticorps de masse moléculaire d'environ 50.000 dalton et possédant une activité de liaison à l'antigène. Il comprend environ la moitié du côté N-terminal de la chaîne lourde et la totalité de la chaîne légère liées par un pont disulfure. Le Fab peut être obtenu notamment par le traitement de l'immunoglobuline par une protéase, la papaïne.

Le terme « *F(ab')2* » désigne un fragment d'environ 100.000 dalton et une activité de liaison à l'antigène. Ce fragment est légèrement plus grand que deux fragments Fab reliés via un pont disulfure dans la région charnière. Ces fragments sont obtenus par traitement d'une immunoglobuline avec une protéase, la pepsine. Le fragment Fab peut être obtenu à partir du fragment F(ab')2 par clivage du pont disulfure de la région charnière.

Une chaîne Fv unique « *scFv* » correspond à un polypeptide VH : VL synthétisé en utilisant les gènes codant pour les domaines VL et VH et une séquence codant pour un peptide destiné à lier ces domaines. Un scFv selon l'invention inclut les CDR maintenus dans une conformation appropriée, par exemple en utilisant des techniques de recombinaison génétique.

Les dimères de « ScFv » correspondent à deux molécules de scFv reliées entre elles par une liaison peptidique. Cette chaîne Fv est fréquemment le résultat de l'expression d'un gène de fusion incluant les gènes codant pour VH et VL liés par une séquence de liaison (linker) codant un peptide. Le fragment scFv humain peut inclure des régions CDRs qui sont maintenues dans une conformation appropriée, préférablement grâce à l'utilisation de techniques de recombinaison génétique. Le fragment "dsFv " est un hétérodimère VH-VL stabilisé par un pont disulfure ; il peut être divalent (dsFV2). Des fragments d'anticorps divalents Sc(Fv)2 ou multivalents peuvent se former spontanément par association de scFvs monovalents ou être produits en reliant des fragments scFvs par des séquences de liaison peptidique.

Le fragment Fc est le support des propriétés biologiques de l'anticorps, en particulier sa capacité à être reconnu par des effecteurs de l'immunité ou à activer le complément. Il est constitué des fragments constants des chaînes lourdes au-delà de la région charnière.

Le terme de "diabodies" signifie de petits fragments d'anticorps ayant deux sites de fixation de l'antigène. Ces fragments comprennent dans la même chaîne polypeptidique VH-VL un domaine variable de chaîne lourde VH connecté à un domaine variable de chaîne légère VL. En utilisant une séquence de liaison qui est trop courte pour permettre l'appariement de deux domaines de la même chaîne, l'appariement avec deux domaines complémentaires d'une autre chaîne se produit nécessairement et ainsi deux sites de fixation d'antigène sont créés.

Un anticorps de l'invention peut ainsi être une immunoglobuline constituée de deux chaînes lourdes et de deux chaînes légères complètes, ou peut être un fragment d'immunoglobuline selon l'invention, par exemple F(ab')2, Fab, Fv, scFv ou Fc. De manière préférée, un tel fragment d'anticorps est la région Fab d'une immunoglobuline, en particulier la région Fv d'un anticorps IgG1.

Les anticorps décrits dans l'invention sont isolés et purifiés, et sont différents des anticorps naturels. Lorsqu'il est sujet d'un anticorps ou d'une séquence nucléotidique selon l'invention, les termes « *isolé* » et « *purifié* » indiquent que la molécule est présente en l'absence majeure d'autres macromolécules biologiques du même type.

Le terme « *anticorps chimérique* » se rapporte à un anticorps dans lequel la séquence de chaque chaîne légère et/ou de chaque chaîne lourde qui le constitue comprend ou consiste en une séquence hybride issue d'au moins deux animaux distincts. De préférence, les anticorps chimériques de l'invention sont des hybrides homme/souris. Notamment, un anticorps chimérique de l'invention peut comprendre un domaine VH et un domaine VL d'un anticorps provenant d'un animal non humain, notamment murin, et un domaine CH et un domaine CL d'un anticorps humain. Ainsi, de manière préférentielle, un anticorps de l'invention comprend un domaine VH et un domaine VL d'un anticorps dérivé de l'anticorps 1G3, défini ci-après, et un domaine CH et un domaine CL d'un anticorps humain.

Selon l'invention, le terme « *anticorps humanisé* » se rapporte à un anticorps issu d'un animal non humain dans lequel les séquences des chaînes lourdes et des chaînes légères autres que les CDRs ont été remplacées par des séquences correspondantes d'un ou plusieurs anticorps d'origine humaine. De manière préférentielle, le terme « anticorps humanisé » se rapporte à un anticorps dont les séquences des chaînes lourdes et des chaînes légères sont d'origine humaine, et dont les CDRs sont issus de l'anticorps 1G3.

Les anticorps selon l'invention sont de préférence des anticorps monoclonaux, c'est-à-dire qu'ils ne reconnaissent qu'un seul déterminant antigénique dans la galectine 9, contrairement aux anticorps polyclonaux qui correspondent à un mélange d'anticorps monoclonaux, qui reconnaissent donc plusieurs déterminants antigénique dans une même molécule.

Des anticorps monoclonaux selon l'invention peuvent être obtenus selon les techniques bien connues de l'homme de métier. Par exemple, il est possible d'utiliser la technique de fusion cellulaire, la technique de clonage des séquences des chaînes lourdes et légères, la technique de phage ou ribosome display, l'immunisation de souris ayant le répertoire des immunoglobulines humaines et expression dans une cellule ad hoc ou un animal transgénique. Ces techniques sont bien connues de l'homme de métier.

La présente invention se rapporte à des anticorps dirigés contre la galectine 9 et inhibiteurs de l'activité suppressive des lymphocytes T régulateurs. En particulier, les inventeurs ont développé un hybridome produisant un anticorps murin IGgl Kappa, 1G3, dirigé contre la galectine 9 et inhibiteur de l'activité suppressive des lymphocytes T régulateurs. Les inventeurs ont caractérisé les domaines variables des chaînes légères et lourdes de cet anticorps monoclonal mAb 1G3 et ont ainsi déterminé les CDRs de cet anticorps, présentés dans le tableau 1.

*Tableau 1*

| Domaines mAb 1G3 | Séquence |
|---|---|
| VH | MKCSWGIFFLLSVTAGVHSKVQLQQSGAELVKPGASVKLSCKAS GYTFTDYTIHWVKQRSGQGLEWIGWFYPGSHSIKYNEQFKDRAT LTADKSSSTVYMELSRLTSEDSAVYFCTRHGGYDGFDYWGQGTT LTVSSAKTTPPSVYPL (**SEQ ID NO:1**) |
| H-CDR1 | GYTFTDYTIH (**SEQ ID NO:2**) |
| H-CDR2 | WFYPGSHSIKYNEQFKDR (**SEQ ID NO:3**) |
| H-CDR3 | HGGYDGFDY (**SEQ ID NO:4**) |
| VL | LDGGKMDSQAQVLMLLLLWVSGTCGDIVMSQSPSSLAVSVGEKI TMSCKSSQSLFYSTNQKNYLAWYQQKPGQSPKLLIYWASTRESG VPDRFTGSGSGTDFTLTISSVKAEDLAVYYCQQYYYFPYTFGGGT KLEIKRADAAPTVSIFPPSS (**SEQ ID NO:5**) |
| L-CDR1 | KSSQSLFYSTNQKNYLA (**SEQ ID NO:6**) |
| L-CDR2 | WASTRES (**SEQ ID NO:7**) |
| L-CDR3 | QQYYYFPYT (**SEQ ID NO:8**) |

Un mode de réalisation particulier de l'invention concerne donc un anticorps dirigé contre la galectine 9 et inhibiteur de l'activité suppressive des lymphocytes T régulateurs, ayant la même zone de fixation que l'anticorps 1G3 ayant pour CDRs les six CDRs définis par :

- la séquence d'acides aminés SEQ ID NO:2 dans la région H-CDR1,

- la séquence d'acides aminés SEQ ID NO:3 dans la région H-CDR2,

- la séquence d'acides aminés SEQ ID NO:4 dans la région H-CDR3,

- la séquence d'acides aminés SEQ ID NO:6 dans la région L-CDR1,

- la séquence d'acides aminés SEQ ID NO:7 dans la région L-CDR2,

- la séquence d'acides aminés SEQ ID NO:8 dans la région L-CDR3.

En particulier, un objet de l'invention concerne un anticorps dirigé contre la galectine 9 et inhibiteur de l'activité suppressive des lymphocytes T régulateurs ayant pour CDRs les six CDRS définis par :

- la séquence d'acides aminés SEQ ID NO:2 dans la région H-CDR1,
- la séquence d'acides aminés SEQ ID NO:3 dans la région H-CDR2,
- la séquence d'acides aminés SEQ ID NO:4 dans la région H-CDR3,
- la séquence d'acides aminés SEQ ID NO:6 dans la région L-CDR1,
- la séquence d'acides aminés SEQ ID NO:7 dans la région L-CDR2,
- la séquence d'acides aminés SEQ ID NO:8 dans la région L-CDR3.

Dans un mode de réalisation particulier, la région variable de chaîne lourde dudit anticorps a la séquence d'acides aminés SEQ ID NO:1 et la région variable de chaîne légère dudit anticorps a la séquence d'acides aminés SEQ ID NO:5.

Un anticorps selon l'invention se lie donc de manière spécifique à la galectine 9 et est inhibiteur de l'activité suppressive des lymphocytes T régulateurs.

Selon un autre mode de réalisation particulier de l'invention, un anticorps selon l'invention peut se lier de manière spécifique à un épitope de la galectine 9. De manière avantageuse, un anticorps selon l'invention est susceptible de se lier de manière spécifique à la galectine 9 membranaire ou intracellulaire

Plus particulièrement encore, un anticorps selon l'invention peut se lier à l'épitope reconnu par l'anticorps 1G3, défini ci-dessus.

Ainsi, selon un mode particulier de l'invention, un anticorps selon l'invention peut se lier de manière spécifique à l'épitope de séquence d'acides aminés SEQ ID NO : 9, présentée dans le tableau 2. Cet épitope consistant en la séquence d'acides aminés SEQ ID NO : 9 correspond au peptide P4 et couvre la fin du peptide de liaison et le début de la partie C-terminale de la galectine 9. Cette séquence existe dans les trois isoformes de la galectine 9 (acides aminés 166 à 178 de l'isoforme S, acides aminés 178 à 190 de l'isoforme M, acides aminés 210 à 222 de l'isoforme L). Un tel anticorps peut donc réagir avec tous les isoformes de la galectine 9.

*Tableau 2*

| Séquence de la galectine 9 (épitope) |
|---|
| TPAIPPMMYPHPA **(SEQ ID NO:9**) |

Selon un autre mode de réalisation particulier, l'anticorps selon l'invention est un anticorps chimérique, de préférence un anticorps chimérique murin/humain. En particulier, cet anticorps chimérique murin/humain peut comprendre les domaines variables de l'anticorps 1G3 comme défini ci-dessus.

Selon un autre mode de réalisation particulier, l'anticorps selon l'invention est un anticorps humanisé. En particulier, le domaine variable de cet anticorps humanisé peut comprendre des régions charpentes de l'accepteur humain, et éventuellement des domaines constants humains, et les CDRs du donneur non-humain, notamment les CDRs définis ci-dessus.

Les inventeurs ont également développé un hybridome produisant un anticorps murin IGg1 Kappa, 2E12, dirigé contre la galectine 9 et inhibiteur de l'activité suppressive des lymphocytes T régulateurs. Les inventeurs ont caractérisé les domaines variables des chaînes légères et lourdes de cet anticorps monoclonal mAb 2E12 et ont ainsi déterminé les CDRs de cet anticorps, présentés dans le tableau 3.

*Tableau 3*

| Domaines mAb 2E12 | Séquence |
|---|---|
| VH | MGWSFIILLSVTAGVHSKVQLQQSGAELVKPGASVKLSCKASGY TFTEYTIHWVKQRSGQGLEWIGWFYPGSGSMEYNEKFDKATLTA DNSSSTVYMELSRLTSEDSAVYFCERHGGYDGFDYWGQGTTLTV SSAKTTPPSVYPLIFLEDLLQYSQLPWKIDVLLLFSQDFQAVY* **(SEQ ID NO:10)** |
| H-CDR1 | GYTFTEYTIH **(SEQ ID NO:11)** |
| H-CDR2 | WFYPGSGSMEYNEKFD **(SEQ ID NO:12)** |
| H-CDR3 | HGGYDGFDY **(SEQ ID NO:13)** |
| VL | LDGGKMDSQAQVLMLLLLWVSGTCGDIVMSQSPSSLAVSVGEK VTMSCKSSQSLLYSNNQKNYLAWYQQKPGQSPKLLIYWASTRGS GVPDRFTGSGSGTDFTLTISSVKAEDLAIYYCQQYYSYPFTFGGGT KLEIKRADAAPTVSIFPPSS **(SEQ ID NO:14)** |
| L-CDR1 | KSSQSLLYSNNQKNYLA **(SEQ ID NO:15)** |
| L-CDR2 | WASTRGS **(SEQ ID NO:16)** |
| L-CDR3 | QQYYSYPFT **(SEQ ID NO:17)** |

Un mode de réalisation particulier de l'invention concerne donc un anticorps dirigé contre la galectine 9 et inhibiteur de l'activité suppressive des lymphocytes T régulateurs, ayant la même zone de fixation que l'anticorps 2E12 ayant pour CDRs les six CDRs définis par :

- la séquence d'acides aminés SEQ ID NO:11 dans la région H-CDR1,
- la séquence d'acides aminés SEQ ID NO:12 dans la région H-CDR2,
- la séquence d'acides aminés SEQ ID NO:13 dans la région H-CDR3,
- la séquence d'acides aminés SEQ ID NO:15 dans la région L-CDR1,
- la séquence d'acides aminés SEQ ID NO:16 dans la région L-CDR2,
- la séquence d'acides aminés SEQ ID NO:17 dans la région L-CDR3.

En particulier, un objet de l'invention concerne un anticorps dirigé contre la galectine 9 et inhibiteur de l'activité suppressive des lymphocytes T régulateurs ayant pour CDRs les six CDRS définis par :

- la séquence d'acides aminés SEQ ID NO:11 dans la région H-CDR1,
- la séquence d'acides aminés SEQ ID NO:12 dans la région H-CDR2,
- la séquence d'acides aminés SEQ ID NO:13 dans la région H-CDR3,
- la séquence d'acides aminés SEQ ID NO:15 dans la région L-CDR1,
- la séquence d'acides aminés SEQ ID NO:16 dans la région L-CDR2,
- la séquence d'acides aminés SEQ ID NO:17 dans la région L-CDR3.

Dans un mode de réalisation particulier, la région variable de chaîne lourde dudit anticorps a la séquence d'acides aminés SEQ ID NO:10 et la région variable de chaîne légère dudit anticorps a la séquence d'acides aminés SEQ ID NO:14.

Comme évoqué précédemment, selon un autre mode de réalisation particulier de l'invention, un anticorps selon l'invention peut se lier de manière spécifique à un épitope de la galectine 9. De manière avantageuse, un anticorps selon l'invention est susceptible de se lier de manière spécifique à la galectine 9 membranaire ou intracellulaire

Plus particulièrement encore, un anticorps selon l'invention peut se lier à l'épitope reconnu par l'anticorps 2E12, défini ci-dessus.

Selon un autre mode de réalisation particulier, l'anticorps selon l'invention est un anticorps chimérique, de préférence un anticorps chimérique murin/humain. En particulier, cet anticorps chimérique murin/humain peut comprendre les domaines variables de l'anticorps 2E12 comme défini ci-dessus.

Selon un autre mode de réalisation particulier, l'anticorps selon l'invention est un anticorps humanisé. En particulier, le domaine variable de cet anticorps humanisé peut comprendre des régions charpentes de l'accepteur humain, et éventuellement des domaines constants humains, et les CDRs du donneur non-humain, notamment les CDRs définis ci-dessus.

Méthodes de production d'anticorps

Les anticorps de l'invention peuvent être produits par n'importe quelle technique connue de l'homme du métier, par exemple mais sans s'y limiter, par toute technique chimique, biologique, génétique ou enzymatique prise seule ou en combinaison.

Il est par exemple possible d'utiliser la technique décrite ci-dessous concernant la fabrication de l'hybridome produisant l'anticorps monoclonal dirigé contre la galectine 9, 1G3 ou 2E12.

La liaison spécifique des anticorps, selon l'invention, dirigés contre la galectine 9 peut être analysée selon n'importe quelle méthode connue de l'état de la technique. Comme immunoessais pouvant être utilisés, on peut par exemple citer les techniques de westerns blots, les tests radio-immunologiques, ELISA, les immunoessais « sandwichs », les tests d'immunoprécipitation, les tests par précipitine, les tests de diffusion sur gel par précipitine, les tests immunoradiométriques, les immunoessais par fluorescence ou les tests de fixation de complément. De tels essais sont bien connus de l'homme du métier.

L'action inhibitrice de l'activité suppressive des lymphocytes T régulateurs d'un anticorps selon l'invention ainsi généré peut être analysée selon différentes techniques connues de l'homme du métier. Par exemple, un test de prolifération cellulaire peut être effectué. On peut ainsi se référer à la technique utilisée ci-dessous concernant le test de prolifération cellulaire effectué avec l'anticorps dirigé contre la galectine 9, 1G3 ou 2E12.

Lorsque la séquence d'acides aminés de la séquence désirée est connue, l'homme du métier peut aisément reproduire l'anticorps par des techniques standards de production de polypeptides.

Par exemple, de tels anticorps peuvent être synthétisés par une méthode bien connue en phase solide, de préférence en utilisant un dispositif de synthèse de peptides disponible dans le commerce et en suivant les recommandations du fournisseur.

De manière alternative, les anticorps de l'invention peuvent être obtenus par des techniques, bien connues de l'homme du métier, d'ADN recombinant dans un système d'expression adapté. Le terme *"système d'expression"* signifie un hôte cellulaire et un vecteur compatible dans des conditions appropriées, c'est-à-dire des conditions permettant l'expression de la protéine codée par l'ADN étranger porté par le vecteur et introduit dans la cellule hôte. Typiquement, la séquence d'acides nucléiques codant un anticorps peut être insérée dans un vecteur d'expression approprié qui sera alors introduit dans un hôte procaryote ou eucaryote adéquat qui produira l'anticorps désiré.

Les termes *"vecteur", "vecteur de clonage"* et *"vecteur d'expression"* se rapportent à des véhicules grâce auxquels les séquences d'ADN ou d'ARN codant l'anticorps peuvent être introduites dans une cellule hôte de façon à la transformer et à permettre l'expression (c'est-à-dire la transcription et la traduction) de la séquence introduite. Un vecteur d'expression est typiquement un plasmide, un cosmide, un épisome, un chromosome artificiel, un phage ou un vecteur viral.

Comme vecteurs viraux, on peut citer les adénovirus, les rétrovirus, le virus de l'herpès et les vecteurs dérivés du virus adéno-associé (AAV). De tels virus recombinants peuvent être produits par des techniques bien connues, comme la transfection de lignées cellulaires permettant leur encapsidation ou par transfection transitoire avec des plasmides ou des virus de complémentation exprimant les fonctions manquantes nécessaires. Des lignées de cellules permettant l'encapsidation sont par exemple PA317, PsiCRIP, GPenv+, 293, etc. Les protocoles détaillés permettant de produire de tels virus recombinants défectueux pour la réplication sont disponibles dans les demandes de brevet WO 95/14785, WO 96/22378, US 5,882,877, etc.

Les cellules hôtes sont donc transfectées, infectées ou transformées par un acide nucléique ou un vecteur approprié tel que décrit ci-dessus.

Le terme de « *transformation* » se rapporte ici à l'introduction d'un gène étranger (extrinsèque ou extracellulaire), d'une séquence d'ADN ou d'ARN dans une cellule hôte de telle manière que cette cellule hôte exprime le gène ou la séquence introduit pour produire la substance désirée, typiquement une protéine codée par le gène ou la séquence introduite.

Des systèmes d'expression usuels incluent, sans limitation, des cellules hôtes et des vecteurs plasmidiques d'E. coli, des cellules hôtes d'insectes et des vecteurs de type Baculovirus et des cellules et des vecteurs de mammifères.

Une méthode de production à partir d'une cellule hôte exprimant un anticorps selon l'invention peut comprendre les étapes consistant à : (i) introduire *in vitro* ou *ex vivo* un acide nucléique recombinant ou un vecteur tel que décrit ci-dessus dans la cellule hôte compétente, (ii) cultiver *in vitro* ou *ex vivo* la cellule hôte recombinante ainsi obtenue, (iii) éventuellement sélectionner les cellules qui expriment et/ou sécrètent ledit anticorps ou polypeptide. De telles cellules hôtes peuvent être utilisées pour la production d'anticorps selon l'invention.

Selon un mode de réalisation particulier, une méthode de production d'un anticorps selon l'invention peut comprendre les étapes consistant à: (i) cultiver la cellule transformée décrite ci-dessus dans des conditions appropriées à l'expression de l'anticorps ; et (ii) récupérer l'anticorps ainsi exprimé.

Les anticorps peuvent être séparés du milieu de culture par des méthodes conventionnelles de purification des immunoglobulines telles que, par exemple, purification sur protéine A-Sepharose, par chromatographie sur hydroxylapatite, par électrophorèse sur gel, par dialyse ou par chromatographie d'affinité.

Dans un mode de réalisation particulier de l'invention, un anticorps chimérique humain selon l'invention peut être produit en obtenant les séquences nucléiques codant les domaines VL et VH comme mentionné précédemment, en construisant un vecteur d'expression d'anticorps chimérique humain par l'insertion des séquences nucléiques dans un vecteur d'expression pour cellule animale ayant des gènes codant les domaines CH et CL, et en exprimant les séquences codées par introduction du vecteur d'expression dans la cellule animale.

Le domaine CH de l'anticorps chimérique humain peut être de n'importe quelle région appartenant à l'immunoglobuline humaine. De préférence, il s'agit de la classe IgG, et de préférence encore IgG1. De la même façon, le domaine CL de l'anticorps chimérique humain peut être de n'importe quelle région appartenant à l'immunoglobuline humaine. De préférence, il s'agit de la classe Kappa.

Les anticorps chimériques ou humanisés selon l'invention peuvent en particulier être obtenus par ingénierie génétique des anticorps.

La construction d'un anticorps chimérique peut par exemple être effectuée par une technique de transfection de gène ou par une technique d'ADN recombinant.

Un anticorps humanisé selon l'invention peut être produit en obtenant les domaines CDRs comme mentionné précédemment, en construisant un vecteur d'expression d'anticorps humain par l'insertion des séquences nucléiques dans un vecteur d'expression pour cellule animale ayant des gènes codant (i) une région constante de chaîne lourde identique à celle d'un anticorps humain et (ii) une région constante de chaîne légère identique à celle d'un anticorps humain, et en exprimant les séquences codées par introduction du vecteur d'expression dans la cellule animale.

En ce qui concerne le vecteur d'expression d'anticorps humanisé, il peut s'agir d'un type dans lequel un gène codant une chaîne lourde d'anticorps et un gène codant une chaîne légère d'anticorps existent dans des vecteurs séparés, soit d'un type dans lequel les deux gènes existent dans le même vecteur (type tandem). Les vecteurs de type tandem sont préférés par rapport à la facilité de construction du vecteur d'expression, la facilité d'introduction dans les cellules animales etc. Comme exemple de vecteur d'expression d'anticorps humanisé de type tandem, on peut citer pKANTEX93 ou pEE18.

Les méthodes de production d'anticorps humanisés basées sur les techniques de transfection de gène ou d'ADN recombinant sont bien connues de l'état de la technique. Les anticorps peuvent être humanisés selon diverses techniques connues de l'état de la technique, par exemple par greffe de CDR (« CDR-grafting »), « veneering » ou « resurfacing », ou encore par réarrangement de chaîne (chain shuffling »). La technique basée sur l'ADN recombinant pour la préparation de tels anticorps est aussi connue.

Un fragment Fab selon l'invention peut être obtenu par traitement d'un anticorps, notamment un anticorps dirigé contre la galectine 9 et inhibiteur de l'activité suppressive des lymphocytes T régulateurs, par une protéase, la papaïne. Ce fragment Fab peut également être produit par insertion d'un ADN codant le fragment Fab de l'anticorps dans un vecteur utilisable dans un système d'expression procaryotique ou eucaryotique et l'introduction de ce vecteur dans le procaryote ou l'eucaryote approprié pour exprimer le fragment Fab.

Un fragment F(ab')2 selon l'invention peut être obtenu par traitement d'un anticorps, notamment un anticorps dirigé contre la galectine 9 et inhibiteur de l'activité suppressive des lymphocytes T régulateurs, par une protéase, la pepsine. Le fragment F(ab')2 peut également être obtenu en joignant entre eux des fragments Fab' tels que décrits ci-dessous, par une liaison thioéther ou un pont disulfure.

Un fragment Fab' selon l'invention peut être obtenu par traitement du complexe F(ab')2 d'un anticorps, notamment un anticorps dirigé contre la galectine 9 et inhibiteur de l'activité suppressive des lymphocytes T régulateurs, par un agent réducteur, le dithiothréitol. Le fragment Fab' peut également être produit par insertion d'un ADN codant le fragment Fab' de l'anticorps dans un vecteur utilisable dans un système d'expression procaryotique ou eucaryotique et l'introduction de ce vecteur dans le procaryote ou l'eucaryote approprié pour exprimer le fragment Fab'.

Le fragment ScFv selon l'invention peut être produit par l'obtention d'une séquence d'ADNc codant les domaines VH et VL précédemment décrits suivie de l'insertion de cet ADN dans un vecteur utilisable dans un système d'expression eucaryotique ou procaryotique et de l'introduction de ce vecteur dans l'eucaryote ou le procaryote approprié pour exprimer le fragment ScFv. Pour obtenir un fragment ScFv humanisé, il est possible d'utiliser la technique de greffe de CDR ("CDR grafting"). Cette technique implique la sélection de régions de complémentarité (CDRs) d'un fragment ScFv donneur et leur greffe sur la charpente d'un fragment ScFv humain de structure tridimensionnelle connue (voir par exemple WO 98/45322; EP0173494).

Des modifications des séquences d'acides aminés des anticorps selon l'invention peuvent être effectuées. Par exemple, il peut être souhaitable d'améliorer l'affinité de liaison et/ou les propriétés biologiques de l'anticorps. Il est connu que, quand un anticorps humanisé est produit par simple greffage des seuls CDRs des VH et VL d'un anticorps dérivé d'un animal non-humain dans les charpentes (FR) d'un anticorps humain, le pouvoir de liaison à l'antigène est diminué en comparaison de celle d'un anticorps d'origine dérivé d'un animal non-humain. Il est considéré que certains résidus d'acides aminés des VH et VL d'un anticorps non-humain, non seulement dans les CDRs mais aussi dans les FRs, sont directement ou indirectement associés au pouvoir de fixation antigénique. La substitution de ces résidus d'acides aminés avec différents résidus d'acides aminés dérivés des FRs des VH et VL de l'anticorps humain réduirait donc le pouvoir de liaison. Par conséquent, afin de résoudre ce problème, des essais doivent être effectués sur les anticorps greffés de CDRs humains afin d'identifier, parmi les séquences d'acides aminés du FR des VH et VL des anticorps humains, un résidu d'acide aminé qui soit directement associé à la liaison avec l'antigène, ou qui interagisse avec un résidu d'acide aminé de CDR ou qui maintienne la structure tridimensionnelle de l'anticorps et qui soit directement associé à la liaison à l'antigène. Le pouvoir de liaison pourrait être augmenté en remplaçant les acides aminés identifiés par les résidus d'acides aminés de l'anticorps d'origine dérivé d'un anticorps non-humain. Des modifications et des changements peuvent être faits dans la structure des anticorps de la présente invention et dans les séquences ADN les codant, tout en obtenant à nouveau une molécule fonctionnelle qui code un anticorps avec les caractéristiques souhaités.

Un autre aspect de la présente invention concerne les variants conservateurs de fonction des anticorps de la présente invention.

Les « *variants conservateurs de fonction* » sont ceux dans lesquels un résidu d'acide aminé donné dans une protéine a été changé sans pour autant altérer la conformation globale et la fonction d'inhibiteur de l'activité suppressive des lymphocytes T régulateurs. Ainsi, il est possible de remplacer un acide aminé par un autre ayant des propriétés similaires (par exemple, polarité, potentiel de liaison hydrogène etc), tant que la fonction d'inhibiteur de l'activité suppressive des lymphocytes T régulateurs est conservée.

Ainsi, selon un mode de réalisation particulier de l'invention, on peut avoir un anticorps tel que précédemment défini, dirigé contre la galectine 9 et inhibiteur de l'activité suppressive des lymphocytes T régulateurs, comprenant:

- une H-CDR1 ayant 1 ou 2 acides aminés de différence avec la séquence définie par SEQ ID NO: 2,

- une H-CDR2 ayant 1 ou 2 acides aminés de différence avec la séquence définie par SEQ ID NO: 3,
- une H-CDR3 ayant 1 ou 2 acides aminés de différence avec la séquence définie par SEQ ID NO: 4,
- une L-CDR1 ayant 1 ou 2 acides aminés de différence avec la séquence définie par SEQ ID NO: 6,
- une L-CDR2 ayant 1 ou 2 acides aminés de différence avec la séquence définie par SEQ ID NO: 7,
- une L-CDR3 ayant 1 ou 2 acides aminés de différence avec la séquence définie par SEQ ID NO: 8.

Selon un autre mode de réalisation particulier de l'invention, un anticorps tel que précédemment défini, dirigé contre la galectine 9 et inhibiteur de l'activité suppressive des lymphocytes T régulateurs, a 1, 2 ou 3 acides aminés de différence avec l'ensemble des six CDRs de séquences tels que précédemment définies, c'est-à-dire les CDRs de séquences SEQ ID NO: 2, SEQ ID NO: 3, SEQ ID NO: 4, SEQ ID NO: 6, SEQ ID NO: 7, et SEQ ID NO: 8.

Selon un autre mode de réalisation particulier de l'invention, on peut avoir un anticorps tel que précédemment défini, dirigé contre la galectine 9 et inhibiteur de l'activité suppressive des lymphocytes T régulateurs, comprenant:

- une H-CDR1 ayant 1 ou 2 acides aminés de différence avec la séquence définie par SEQ ID NO: 11,
- une H-CDR2 ayant 1 ou 2 acides aminés de différence avec la séquence définie par SEQ ID NO: 12,
- une H-CDR3 ayant 1 ou 2 acides aminés de différence avec la séquence définie par SEQ ID NO: 13,
- une L-CDR1 ayant 1 ou 2 acides aminés de différence avec la séquence définie par SEQ ID NO: 15,
- une L-CDR2 ayant 1 ou 2 acides aminés de différence avec la séquence définie par SEQ ID NO: 16,
- une L-CDR3 ayant 1 ou 2 acides aminés de différence avec la séquence définie par SEQ ID NO: 17.

Selon un autre mode de réalisation particulier de l'invention, un anticorps tel que précédemment défini, dirigé contre la galectine 9 et inhibiteur de l'activité suppressive des lymphocytes T régulateurs, a 1, 2 ou 3 acides aminés de différence avec l'ensemble des six CDRs de séquences tels que précédemment définies, c'est-à-dire les CDRs de séquences SEQ ID NO: 11, SEQ ID NO: 12, SEQ ID NO: 13, SEQ ID NO: 15, SEQ ID NO: 16, et SEQ ID NO: 17.

Utilisations thérapeutiques

Comme mentionné précédemment, il est connu qu'en situation pathologique, les lymphocytes T régulateurs peuvent induire une immunosuppression inappropriée, qui favorise alors la croissance tumorale. De nombreuses études ont ainsi montré que les lymphocytes T régulateurs diminuent les réponses immunitaires anti-tumorales, notamment en inhibant de manière inappropriée l'activité des lymphocytes T effecteurs, favorisant ainsi le développement de pathologies de type cancers.

Il a ici été démontré que, d'une part, lors de l'activation, la galectine 9 est directement exprimée par les lymphocytes T régulateurs tandis qu'elle ne l'est que très faiblement, voire pas du tout, par les lymphocytes T effecteurs, le ciblage de la galectine 9 permettant d'inhiber de manière spécifique les lymphocytes T régulateurs sans risquer de provoquer une déplétion de lymphocytes T effecteurs. Il a d'autre part été démontré que l'inhibition de la galectine 9 par un anticorps permet l'inhibition de l'activité suppressive des lymphocytes T régulateurs. Les anticorps selon l'invention, dirigés contre la galectine 9 et inhibiteurs de l'activité suppressive des lymphocytes T régulateurs, peuvent donc être utilisés dans le traitement de maladies associées à l'activité suppressive des lymphocytes T régulateurs, notamment le traitement de cancers.

Un objet de l'invention se rapporte ainsi à un anticorps tel que précédemment décrit pour son utilisation dans le traitement de maladies associées à l'activité suppressive des lymphocytes T régulateurs.

Un mode de réalisation particulier de l'invention se rapporte à un anticorps, tel que précédemment décrit, utilisé dans le traitement de cancer.

On entend par « *traitement de cancer* » tout traitement capable, par exemple, de supprimer une tumeur ou des métastases, réduire le risque de récidive, ralentir le développement d'une tumeur ou des métastases, et/ou traiter les symptômes de la maladie.

Les cancers visés par la présente invention sont ceux dans lesquels les lymphocytes T régulateurs exercent leur activité suppressive. De manière avantageuse, les cancers visés par la présente invention sont ceux dans lesquels les lymphocytes T régulateurs sont présents en grande quantité dans le tissu tumoral ou dans la circulation, l'expansion des lymphocytes T régulateurs étant généralement corrélée à l'augmentation de leur activation (6). La fréquence des lymphocytes T régulateurs peut être évaluée par toute méthode connue de l'homme du métier, par exemple par une analyse en cytométrie de flux (FACS) des lymphocytes intra-tumoraux ou des lymphocytes circulants ou par un marquage immuno-histologique du tissu tumoral.

De manière générale, un anticorps tel que précédemment défini peut donc être utilisé dans le traitement de tous types de cancers dans lesquels les lymphocytes T régulateurs exercent leur activité suppressive. De nombreux types de cancers dans lesquels les lymphocytes T régulateurs exercent leur activité suppressive ont faits l'objet d'études et sont connus de l'homme du métier.

Il est ainsi connu que des taux élevés de lymphocytes T régulateurs au sein des tumeurs sont clairement associés à un mauvais pronostic dans les Leucémies Myéloïdes Chroniques (7), le cancer du colon (8), le mélanome (9), le cancer de l'utérus (10), le cancer du sein (11), le cancer du pancréas (12), les cancers gastriques (13), le cancer des ovaires (14), le lymphome primaire du système nerveux central (15), les myélomes multiples (16), le cancer de la prostate (17), le lymphome de Hodgkin ou le carcinome hépatocellulaire (18, 19).

Un anticorps tel que précédemment défini peut donc en particulier être utilisé dans le traitement de cancer, le cancer étant choisi dans le groupe constitué des Leucémies Myéloïdes Chroniques, cancer du colon, mélanome, cancer de l'utérus, cancer du sein, cancer du pancréas, cancers gastriques, cancer des ovaires, lymphome primaire du système nerveux central, myélomes multiples, cancer de la prostate, lymphome de Hodgkin et carcinome hépatocellulaire.

Des études ont également démontré que certains cancers produisent des quantités importantes d'exosomes porteurs de la galectine 9 jouant un rôle immunosuppresseur, c'est-à-dire inhibant la réponse immune et potentiellement la réponse anti-tumorale. On peut citer comme exemples, non limitatifs, de cancers produisant des quantités importantes d'exosomes porteurs de la galectine 9, les cancers viro-induits, par exemple les carcinomes du nasopharynx associés au Virus d'EBV (virus Epstein-Barr) ou les carcinomes hépatocellulaires (CHC) liés au VHC (virus de l'hépatite C) ou VHB (virus de l'hépatite B) (20,21).

Un anticorps tel que précédemment défini peut donc en particulier être utilisé dans le traitement de cancer, le cancer étant un cancer viro-induit, de préférence choisi dans le groupe constitué des carcinomes du nasopharynx associés au Virus d'Epstein-Barr, des carcinomes hépatocellulaires liés au virus de l'hépatite C ou au virus de l'hépatite B.

Il a également été démontré que l'augmentation de la fréquence des lymphocytes T régulateurs est un facteur prédictif de la récidive d'une fibrose consécutive à une hépatite C (22,23).

Un anticorps selon l'invention peut donc être utilisé en prévention d'une récidive d'une fibrose consécutive à une hépatite C.

Dans chacun des modes de réalisation précédemment décrits, l'anticorps dirigé contre la galectine 9 et inhibiteur de l'activité suppressive des lymphocytes T régulateurs est administré d'une manière appropriée à un patient nécessitant un tel traitement.

Les anticorps selon l'invention peuvent être utilisés seuls ou en combinaison avec n'importe quel autre composé approprié.

Un objet de l'invention se rapporte à une méthode de traitement d'un cancer, associé à l'expression de la galectine 9 et à l'activité suppressive des lymphocytes T régulateurs, comprenant l'administration d'une quantité thérapeutiquement active d'un anticorps selon l'invention à un patient.

Le terme « *patient* » est entendu comme un humain affecté, ou amené à être affecté, par une maladie associée à l'activité suppressive des lymphocytes T régulateurs, notamment un cancer.

Le terme « *quantité thérapeutiquement active* » d'un anticorps, signifie une quantité d'anticorps suffisante pour traiter un tel cancer, ayant un rapport bénéfice/risque acceptable pour un traitement médicamenteux. La quantité d'anticorps et de compositions selon la présente invention ainsi que la fréquence d'administration sera déterminée par des études cliniques, par le médecin ou par le pharmacien. La quantité "thérapeutiquement active" spécifique à chacun des patients pourra dépendre d'un certain nombre de facteurs comme la nature et la sévérité du désordre à traiter, l'activité de l'anticorps utilisé, la composition utilisée, l'âge, le poids, l'état de santé général, le sexe et le régime du patient, le mode d'administration, la durée du traitement (en monodose ou en plusieurs doses), les médicaments utilisés en combinaison et d'autres facteurs bien connus des spécialistes médicaux.

Selon un mode de réalisation particulier de l'invention, un anticorps dirigé contre la galectine 9 et inhibiteur de l'activité suppressive des lymphocytes T régulateurs tel que précédemment défini est utilisé en combinaison avec un deuxième agent pour le traitement d'une maladie associée à l'activité suppressive des lymphocytes T régulateurs, par exemple un agent anticancéreux.

Ainsi, quand l'utilisation est le traitement d'un cancer, l'anticorps peut être utilisé en combinaison avec des thérapies connues contre le cancer comme par exemple, la chirurgie, radiothérapie, chimiothérapie ou des combinaisons de celles-ci. Par exemple, l'anticorps peut être utilisé en combinaison avec une immunothérapie adoptive, consistant en une ou plusieurs injections de lymphocytes effecteurs contre des antigènes tumoraux notamment des antigènes d'EBV. Selon certains aspects, d'autres agents anticancéreux utilisés dans la combinaison avec l'anticorps dirigé contre la galectine 9 selon l'invention pour la thérapie du cancer comprennent les agents anti-angiogéniques. Selon certains aspects, ledit anticorps est co-administré avec une cytokine, par exemple une cytokine qui stimule une réponse immunitaire anti-tumorale.

Un objet de l'invention se rapporte donc à un produit de combinaison comprenant un anticorps, tel que précédemment défini, et un agent anticancéreux.

Un mode de réalisation particulier se rapporte à un tel produit de combinaison pour une utilisation simultanée, séparée ou étalée dans le temps dans le traitement de cancer.

Compositions pharmaceutiques

Pour l'administration, l'anticorps est en général formulé sous forme de composition pharmaceutique. La composition pharmaceutique comprenant un anticorps selon l'invention peut être formulée par des méthodes connues de l'état de la technique, dans lesquelles la molécule thérapeutique est en combinaison avec au moins un excipient.

Un objet de l'invention se rapporte ainsi à une composition pharmaceutique comprenant un anticorps tel que précédemment décrit et au moins un support pharmaceutiquement acceptable.

Un « *support pharmaceutiquement acceptable* » s'entend par tout support pharmaceutique standard, son administration pouvant être tolérée par un patient. Par exemple, des solutions salines tamponnées au phosphate et stériles sont pharmaceutiquement acceptables.

Les supports pharmaceutiquement acceptables peuvent habituellement comprendre un ou plusieurs composés, par exemple choisis parmi des excipients, conservateurs, solubilisants, agents tampon, albumine etc. Des excipients connus sont par exemple de l'amidon, gélatine, acide stéarique, stéarate de calcium ou de magnésium etc. L'homme du métier saura déterminer les composés adaptés à la présente composition.

La forme de la composition pharmaceutique, le mode d'administration, le dosage et la posologie peuvent bien sûr dépendre, entre autres, de la maladie à traiter, de ses symptômes, de sa sévérité, de l'âge, du poids et du sexe du patient.

De façon non limitative, la composition pharmaceutique selon l'invention peut être formulée de façon à pouvoir être administrée par voie topique, parentérale, nasale, intraveineuse, sous-cutanée/intra-dermique, conjonctivale, intramusculaire ou intraoculaire.

Les compositions pharmaceutiques selon l'invention, peuvent éventuellement contenir des excipients pharmaceutiquement acceptables appropriés pour qu'elles puissent être injectées. En particulier ceux-ci peuvent être des solutions salines isotoniques et stériles, phosphate monosodique ou disodique, chlorure de sodium, potassium, calcium ou magnésium, etc., ou mélange de ces sels. Ces compositions peuvent également être des compositions sèches, en particulier des compositions sèches et congelées, lyophilisées ou réfrigérées, qui après addition, selon les cas, d'eau stérile ou d'eau physiologique, constituent des solutions injectables.

Les doses utilisées peuvent être adaptées en fonction de différents paramètres comme, en particulier, le mode d'administration selon la pathologie ou alternativement, la durée du traitement envisagée.

Pour préparer les compositions pharmaceutiques, une quantité suffisante d'anticorps peut être dissoute ou dispersée dans un véhicule pharmaceutiquement acceptable ou un milieu aqueux.

Les formes pharmaceutiques appropriées à une utilisation par injection comprennent les solutions d'eau stérile, les dispersions, les formulations incluant de l'huile de sésame, ou du propylène glycol aqueux, ainsi que des poudres stériles pour la préparation extemporanée de solutions injectables stériles. Dans tous les cas, la forme utilisée doit être stérile et doit être suffisamment fluide pour pouvoir être injectée facilement au moyen d'une seringue. Elle doit être stable dans les conditions de production et de stockage et être protégée des contaminations par des microorganismes, tels que les bactéries ou les champignons.

Les solutions des composés actifs, qu'ils soient sous forme libre ou en tant que sels acceptables d'un point de vue pharmaceutique, peuvent être préparées avec de l'eau mélangée à un surfactant comme l'hydroxypropylcellulose. Les dispersions peuvent être faites dans du glycérol, dans des polyéthylènes glycols liquides, dans un mélange des deux ou dans des huiles. Ces préparations contiennent généralement un agent conservateur pour empêcher la croissance de micro-organismes dans les conditions normales de stockage et d'utilisation.

Un anticorps selon l'invention peut être formulé dans une composition sous forme neutre ou sous forme de sel. Des sels pharmaceutiquement acceptables comprennent les sels d'addition acides, formés avec les groupements aminés libres de la protéine, et formés avec des acides inorganiques comme par exemple les acides hydrochloriques ou phosphoriques, ou des acides organiques comme les acétique, oxalique, mandélique, etc. Les sels formés avec les groupements carboxyl libres peuvent aussi être dérivés de bases inorganiques comme le sodium, potassium, ammonium, calcium, ou les hydroxydes de fer, et de bases organiques comme l'isopropylamine, trimethylamine, histidine, procaine etc.

Après formulation sous forme de médicament, les solutions peuvent être administrées d'une manière compatible avec le dosage de la formulation et en une quantité thérapeutiquement active. Les médicaments peuvent être administrées comme décrit ci-dessus mais également sous forme de capsules qui les libèrent.

**FIGURES**

**Figure 1 :** Analyse phénotypique par cytométrie de flux multiparamétrique des lymphocytes T régulateurs naturels isolés du sang humain.

**Figure 2** : Analyse de la fonction suppressive des lymphocytes T régulateurs naturels isolés *ex vivo*. (A) Analyse de l'inhibition de la prolifération des PBMCs activés par les lymphocytes T régulateurs autologues en cpm. (B) Analyse du pourcentage de cytolyse des PBMCs activés par les lymphocytes T régulateurs autologues.

**Figure 3** : Analyse par Q-PCR de l'expression du gène codant la galectine 9 dans les lymphocytes T régulateurs humains (n=12). 1) 8, D1, D2, A1, A2 représentent les poches de sang. CT (Cycle Threshold) représente le seuil de cycle. Il s'agit du seuil moyen à partir duquel on commence à détecter l'amplification du gène de la Galectine 9. Les résultats sont exprimés en ΔCT suite à la normalisation avec quatre gènes de ménages (β-Actin, GAPDH, HPRT, Ubiquitin)
[ΔCT= CT (l'échantillon) - CT (moyenne des gènes de ménage)]

**Figure 4** : Analyse par western blot de l'expression des trois isoformes de la galectine 9 dans les lymphocytes T régulateurs humains. Les cellules Hela sont utilisées comme contrôle négatif.

**Figure 5** : Analyse par Q-PCR de l'expression du gène codant la galectine 9 dans les lymphocytes T conventionnels au cours de l'activation (n=4).

**Figure 6** : Analyse par Q-PCR de l'expression du gène codant la galectine 9 dans les lymphocytes T régulateurs humains au cours de l'activation (n=4).

**Figure 7 :** Analyse de l'inhibition de l'activité suppressive des lymphocytes T régulateurs par l'anticorps anti-gal9 1G3 par l'analyse de la prolifération des PBMCs en présence des C15 irradiées, en présence ou non de lymphocytes T régulateurs, et en présence ou non d'anticorps 1G3 à la concentration de 1μg/mL.

**Figure 8** : Analyse de l'inhibition de l'apoptose de Jurkat induite par la galectine 9 Avec 1h de préincubation de Galectin-9 S à 1 μg / mL et des anticorps à 5 μg / ml (anticorps 9M1 (anti-galectine 9), 9S2-3 (anti-galectine 9), 1G3 (anti-galectine 9), un anticorps anti-TIM3, 2E12 (anti-galectine 9)).

**Figure 9** : Analyse de la restauration de la prolifération sur cellules activées (« A », avec de l'anti-CD3 et de l'anti-CD28) et non-activées (NA), après traitement avec de la galectine 9 (anticorps testés = ECA-42 (anti-galectine 9), 1G3 (anti-galectine 9), 2E2 (anti-TIM3) et 2E12 (anti-galectine 9). Le contrôle correspond au milieu de culture.

**Figure 10** : Analyse de l'expression de la galectine 9 par cytométrie de flux (FACS).

A : Expression de la galectine 9 à partir de lymphocytes T régulateurs et de cellules T CD4+ conv fraichement isolés (n= 2 donneurs).
B : Expression de la galectine 9 extrait de lymphocytes T régulateurs et de cellules T CD4+ conv après 24h d'activation (n=5 donneurs).
C : Expression de la galectine 9 extrait de lymphocytes T régulateurs et de cellules T CD4+ conv après 48h d'activation (n=5 donneurs).
D : Expression de la galectine 9 extrait de lymphocytes T régulateurs et de cellules T CD4+ conv après 72h d'activation (n=5 donneurs).

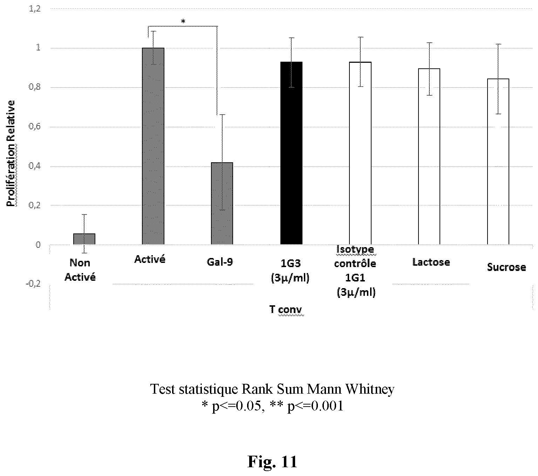

**Figure 11:** Prolifération relative des lymphocytes T conv sous différentes conditions après 3 jours de culture (cellules activées ou non, en présence de galectine 9 et avec 1G3 (« anti-X »), un isotype contrôle (IgG1), un inhibiteur (lactose) et un inhibiteur témoin (sucrose)).

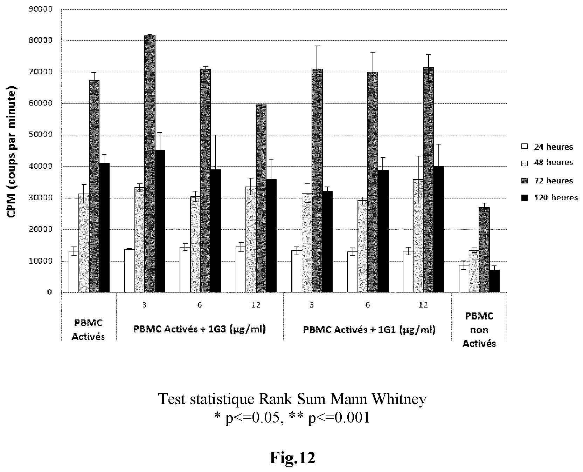

**Figure 12** : Prolifération des PBMC sous différentes conditions après 5 jours de culture (cellules activées ou non, en présence de 1G3 ou d'un isotype contrôle (IgG1).

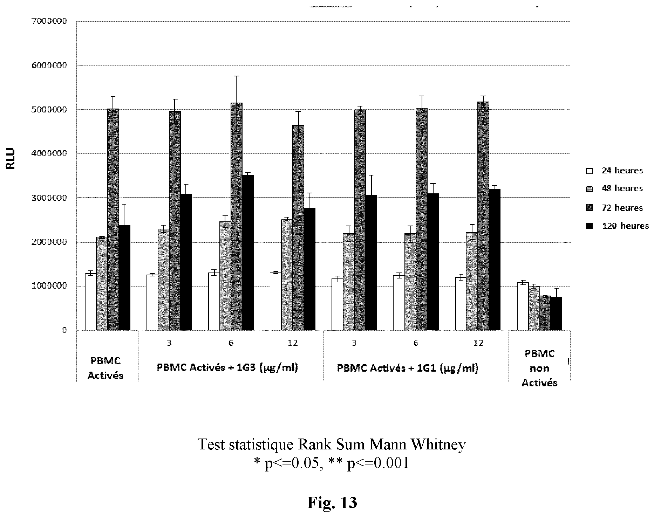

**Figure 13** : Viabilité des PBMCs sous différentes conditions après 5 jours de culture (cellules activées ou non, en présence de 1G3 ou d'un isotype contrôle (IgG1).

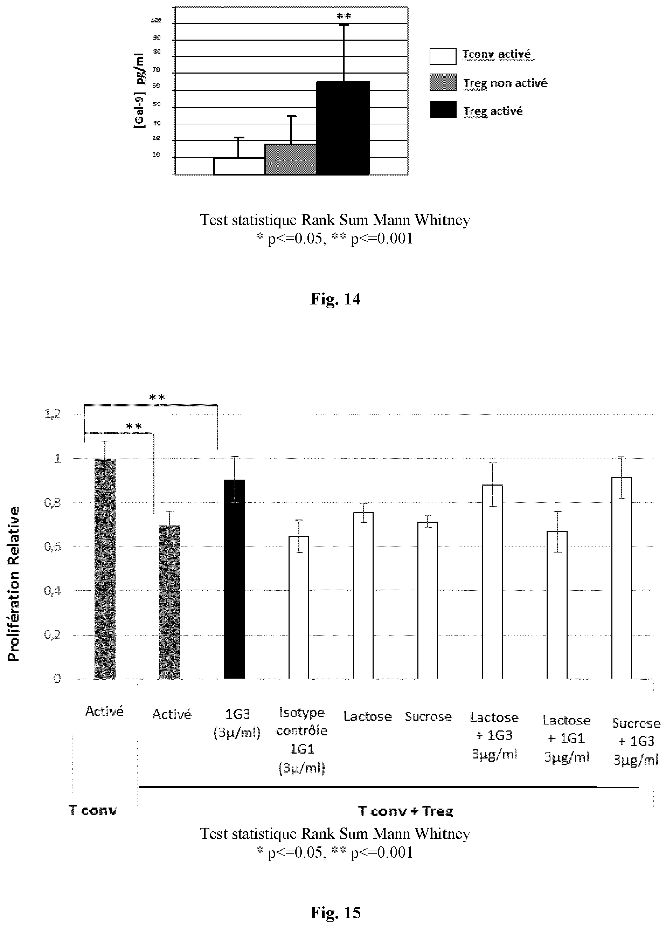

**Figure 14** : Mesure de la sécrétion de la galectine 9 par les T CD4+ conventionnels (« T conv ») et les lymphocytes T régulateurs (« Tregs ») en conditions non-activées et activées (test réalisé après 48h de culture).

**Figure 15 :** Prolifération relative des lymphocytes T conventionnels en co-culture avec des lymphocytes T régulateurs autologues sous différentes conditions (cellules activées, en présence de 1G3 (« anti-X »), un isotype contrôle (IgG1), un inhibiteur (lactose) et/ou un inhibiteur témoin (sucrose)).

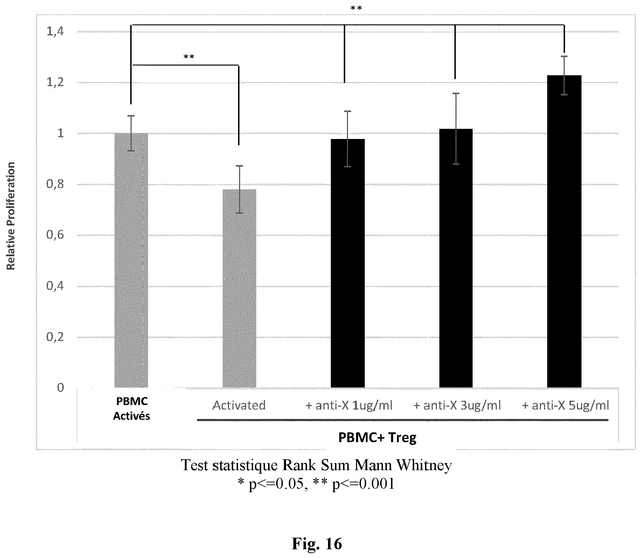

**Figure 16** : Prolifération relative des PBMCs en présence de lymphocytes T régulateurs et de différentes concentrations de 1G3 (1 $\mu$/ml, 3 $\mu$/ml, 5 $\mu$/ml).

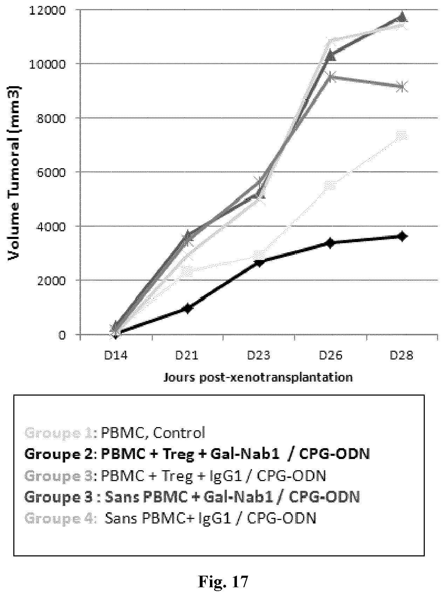

**Figure 17 :** Volume tumoral sous différents traitements : PBMCs seules (contrôle) ; PBMC + Treg + 1G3 (activés) ; PBMC + Treg + IgG1 (activés) ; sans PBMC + 1G3 (activés) ; sans PBMC+ IgG1 (activés)

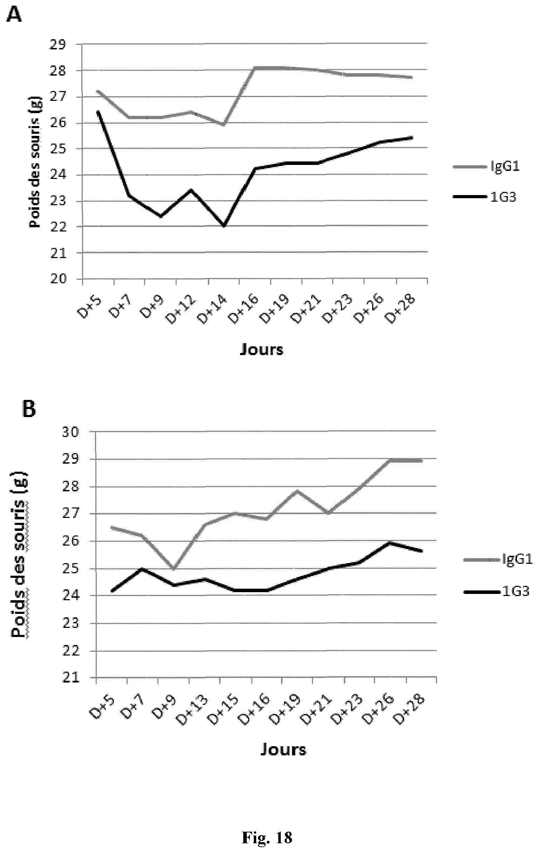

**Figure 18** : Représentation graphique du poids des souris en fonction des jours. A et B : Expériences indépendantes avec souris SCID xénotransplantées ($50.10^6$ PBMCs + 6% de Treg) et traitées avec 1G3 ou l'isotype contrôle IgG1 (20 $\mu$g/souris).

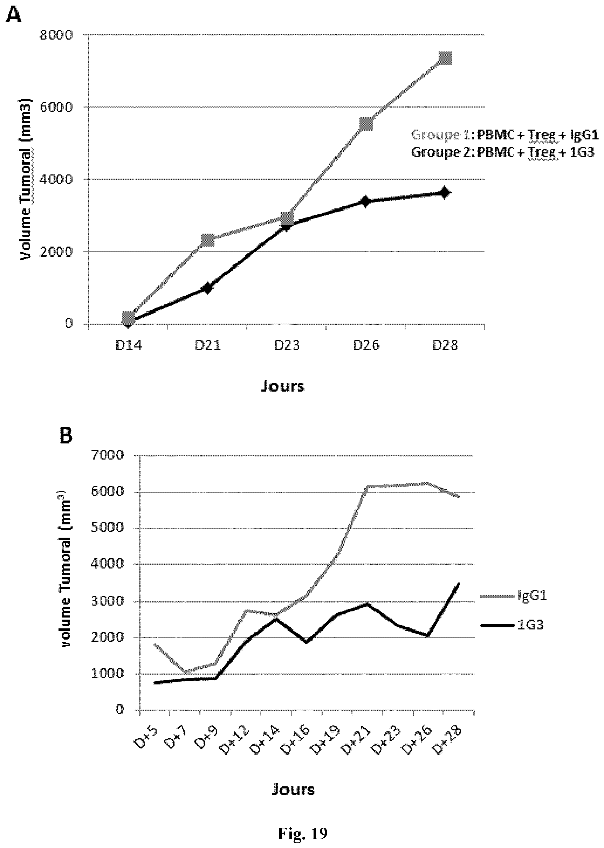

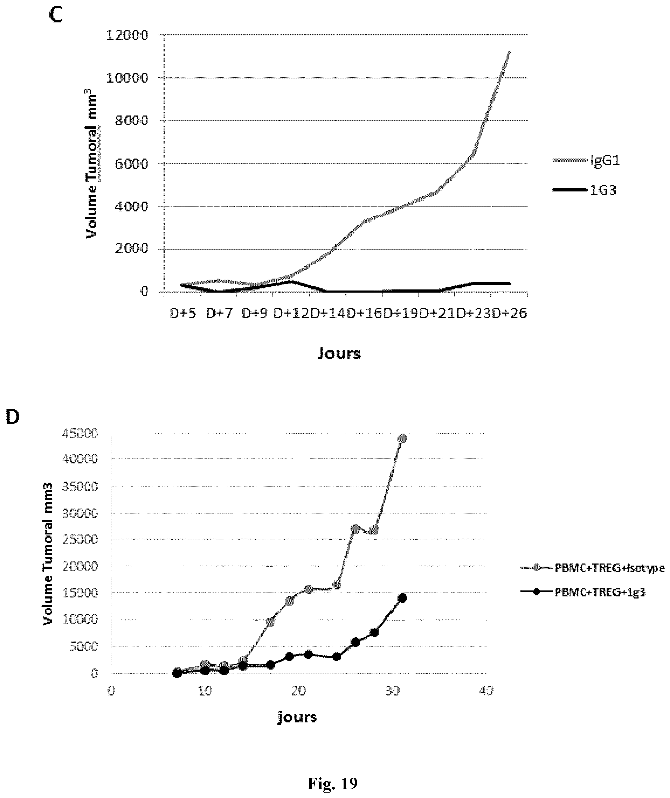

**Figure 19** : Représentation graphique du poids des souris en fonction des jours.

A : Souris SCID xénotransplantées ($20.10^6$ PBMCs + 10% de Treg) et traitées avec 1G3 ou l'isotype contrôle IgG1 (2 $\mu$g/souris).
B : Souris SCID xénotransplantées ($50.10^6$ PBMCs + 6% de Treg) et traitées avec 1G3 ou l'isotype contrôle IgG1 (20 $\mu$g/souris).
C : Souris SCID xénotransplantées ($50.10^6$ PBMCs + 6% de Treg) et traitées avec 1G3 ou l'isotype contrôle IgG1 (20 $\mu$g/souris).
D : Souris SCID xénotransplantées ($50.10^6$ PBMCs + 6% de Treg) et traitées avec 1G3 ou l'isotype contrôle IgG1 (200 $\mu$g/souris).

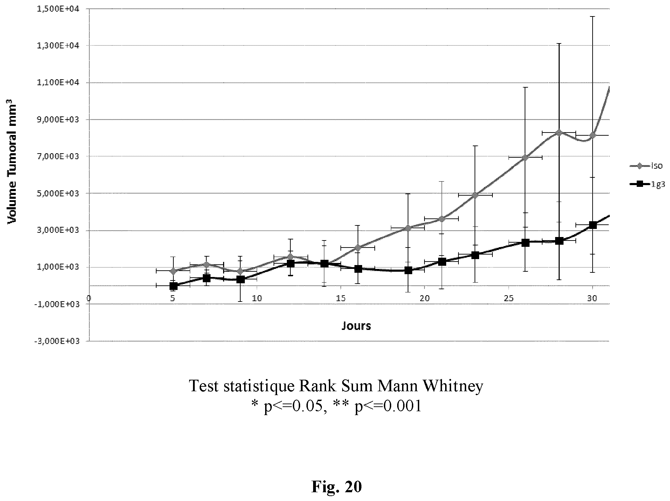

**Figure 20 :** Moyennes du volume tumoral mesuré manuellement ($mm^3$) de 6 expériences indépendantes (20 $\mu$g de 1G3 par souris, $50.10^6$ PBMC + 6 à 8% de Treg, en comparaison à l'isotype contrôle IgG1) (mesure manuelle). (*p<=0.05, **p<=0.001)

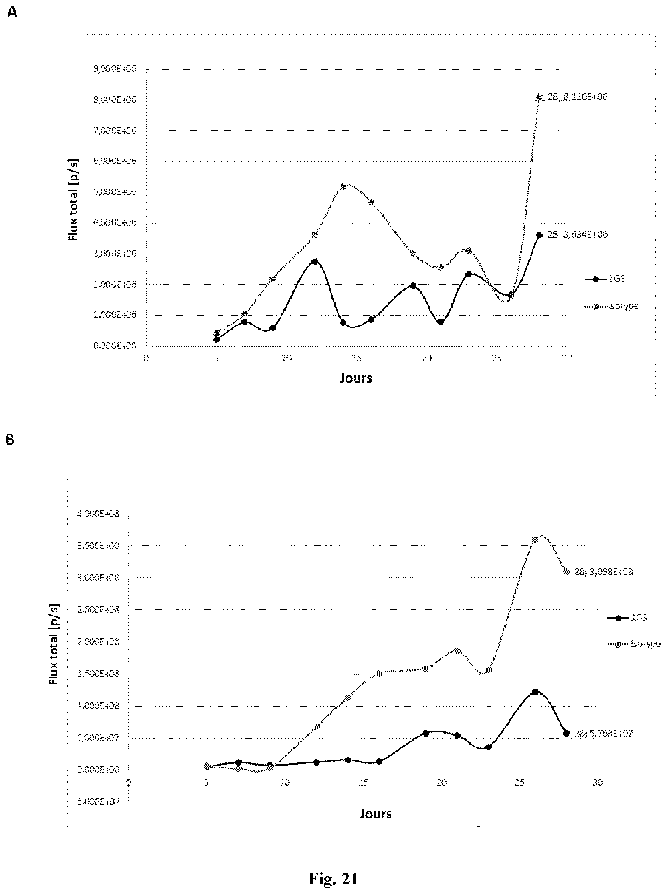

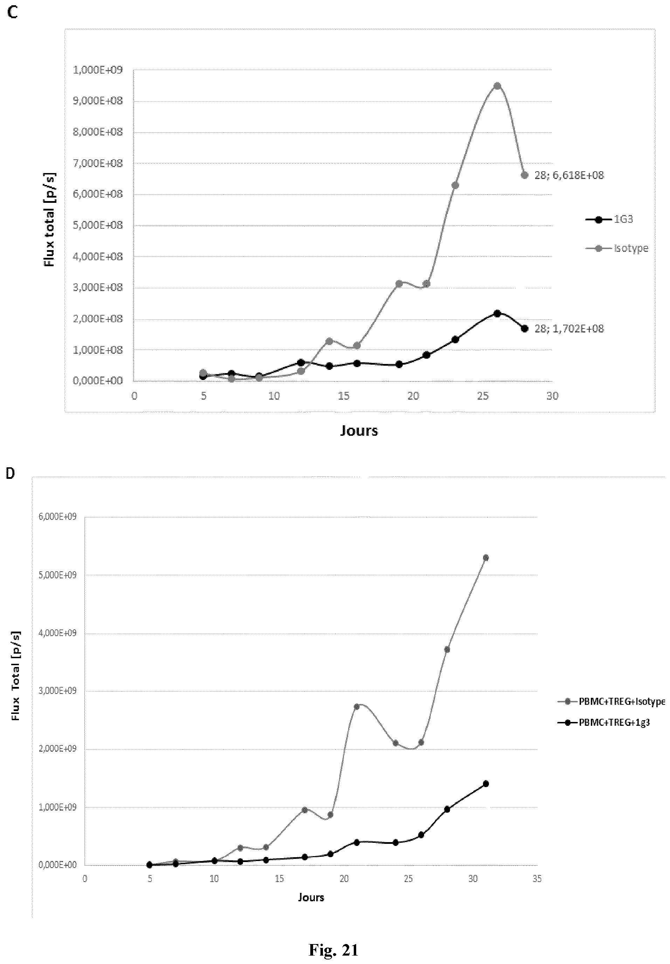

**Figure 21** : Mesures du volume tumoral mesuré par bioluminescence.

A : Souris SCID xénotransplantées ($50.10^6$ PBMCs + 6% de Treg) et traitées avec 1G3 ou l'isotype contrôle IgG1 (20 $\mu$g/souris).
B : Souris SCID xénotransplantées ($50.10^6$ PBMCs + 6% de Treg) et traitées avec 1G3 ou l'isotype contrôle IgG1 (20 $\mu$g/souris).
C : Souris SCID xénotransplantées ($40.10^6$ PBMCs + 6% de Treg) et traitées avec 1G3 ou l'isotype contrôle IgG1 (20 $\mu$g/souris).
D : Souris SCID xénotransplantées ($50.10^6$ PBMCs + 6% de Treg) et traitées avec 1G3 ou l'isotype contrôle IgG1 (200 $\mu$g/souris).

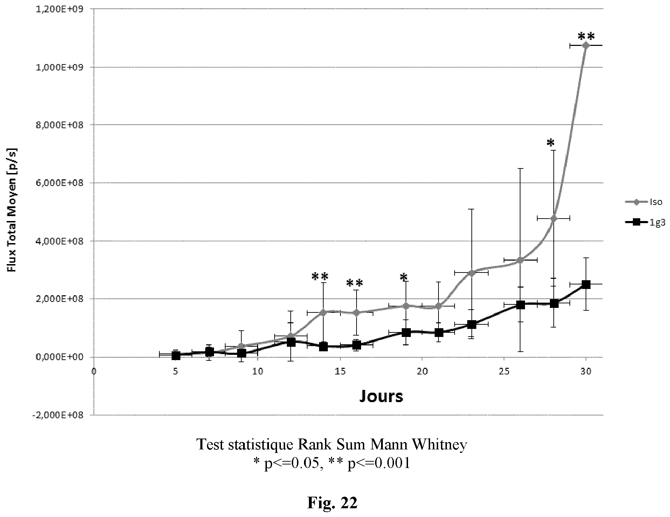

**Figure 22 :** Moyennes du volume tumoral mesuré par Bioluminescence de 6 expériences indépendantes (20 $\mu$g de 1G3 ou IgG1 par souris, $50.10^6$ PBMC + 6 à 8% de Treg) (mesure du Flux Total). (*p<=0.05, **p<=0.001)

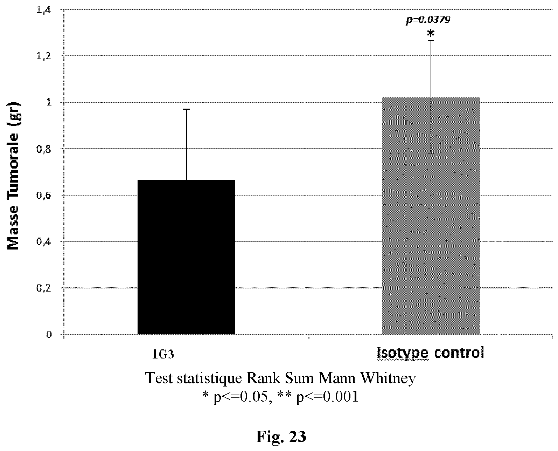

**Figure 23** : Masses tumorales en grammes moyennée sur 6 expériences indépendantes (20 $\mu$g de 1G3 ou IgG1 par souris, $50.10^6$ PBMC + 6 à 8% de Treg). (*p<=0.05, **p<=0.001)

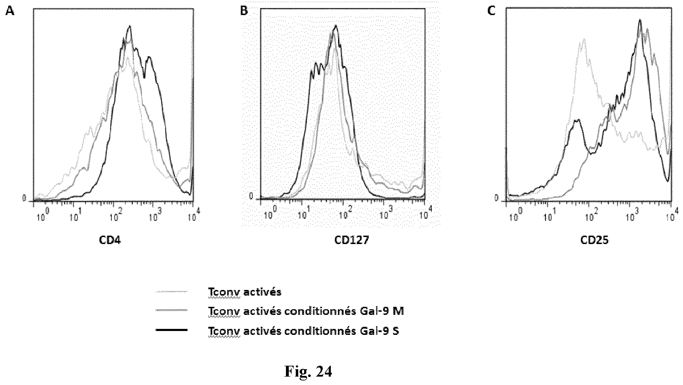

**Figure 24** : Analyse cytométrique comparative de l'expression de différents marqueurs après 5 jours de conditionnement. A : Expression du CD4 entre Tconv activés (gris clair), activés avec la Gal-9 M (gris moyen) ou la Gal-9-S (noir). B : Expression du CD127 entre Tconv activés (gris clair), activés avec la Gal-9 M (gris moyen) ou la Gal-9- S (noir).. C : Expression du CD25 entre Tconv activés (gris clair), activés avec la Gal-9 M (gris moyen) ou la Gal-9- S (noir).

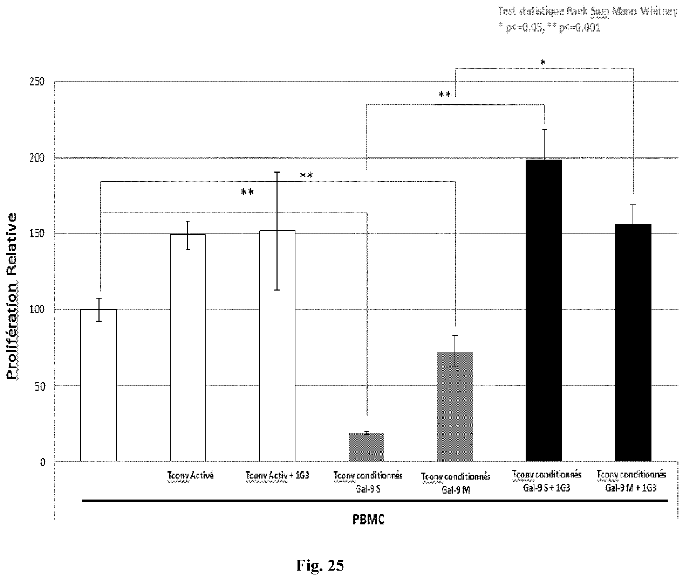

**Figure 25** : Prolifération relative des PBMCs en fonction de diverses conditions après 3 jours de culture avec des Tconv conditionnés par la forme M ou S de la Gal-9 pendant 5 jours. (*p<=0.05, **p<=0.001)

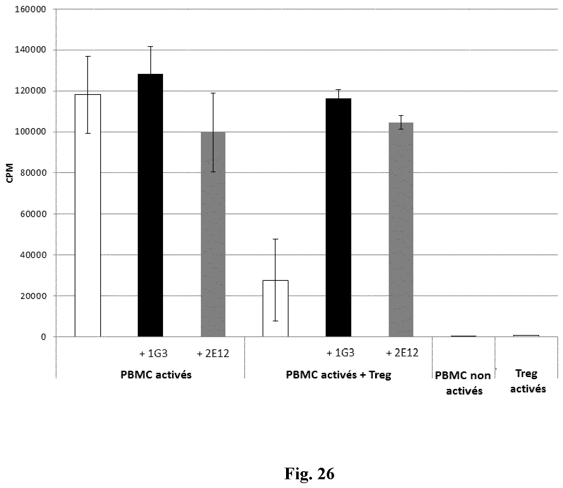

**Figure 26 :** Analyse de l'inhibition de l'activité suppressive des lymphocytes T régulateurs par l'anticorps anti-gal9 1G3 et l'anticorps anti-gal9 2E12 (à la concentration de 1$\mu$g/mL), par l'analyse de la prolifération des PBMCs co-cultivés avec des lymphocytes T régulateurs.

## EXEMPLES

### 1. Matériels et méthodes

**Donneurs, lignées cellulaires et conditions de culture**

*Cellules de donneurs*

Les cellules de donneurs sains ont été isolées à partir de sang provenant de l'Etablissement Français du Sang - Nord de France (EFS), conformément à un accord officiel d'éthique entre ce dernier et le Centre National de la Recherche Scientifique (CNRS) - Délégation Nord Pas-de-Calais et Picardie. L'étude a été approuvée par l'Institut de Biologie de Lille (CNRS) et le comité institutionnel de l'EFS, et chacun des donneurs avait préalablement signé un consentement éclairé.

*Lignées cellulaires de CNP*

Les lignées cellulaires tumorales C15 sont dérivées d'un CNP (Carcinome du Nasopharynx) EBV-positif xénotransplanté et propagé en continu dans des souris SCID en sous-cutané tous les 6-7 semaines. Toutes les expérimentations animales ont été réalisées par un personnel qualifié, conformément aux règlements Français et Européens, au sein de l'animalerie de l'Institut Pasteur de Lille (France). Les cellules de C15 ont été récupérées à partir des tumeurs xénotransplantées, puis irradiées (5000 rads) avant d'être pré-incubées avec les cellules immunitaires simulant un contexte tumoral.

*Lignée lymphocytaire T humaine Jurkat*

La lignée Jurkat a été établie à partir d'une leucémie lymphocytaire T humaine. Elle présente le phénotype de lymphocytes CD4+.

*Conditions des cultures cellulaires*

Le milieu de culture standard utilisé est le RPMI 1640 (Invitrogen, Paisley, UK) complété avec 10% de sérum humain AB (BioWest, Nuaillé, France), 2 mM de L Glutamine, 1 mM de sodium pyruvate, 10 mM d'acides aminés non-essentiels, 10 mM d'HEPES, 50 U/mL de streptomycine, 50 $\mu$g/mL de gentamycine et 50 $\mu$M $\beta$ mercaptoethanol. Les cellules ont été incubées à 37°C sous une atmosphère contrôlée (5% de $CO_2$ et 95% d'humidité) dans un incubateur Hera Cell 150 (Thermo Electron, Cergy Pontoise, France). Le cas échéant, les PBMCs et les cellules T CD4+ ont été activées avec un anticorps anti CD3 (1 $\mu$g/mL) (Clinisciences, Montrouge, France), qui se fixe à la plaque après une incubation de 2 heures à 37°C avant la culture, et un anticorps soluble anti CD28 (100 ng/mL) (Clinisciences) ajouté extemporanément.

**Isolement des cellules immunitaires humaines**

*Isolement des PBMCs*

Les cellules mononuclées du sang périphérique (PBMCs) de donneurs sains ont été isolées par la centrifugation en gradient de densité standard utilisant le Ficoll Paque PLUS (Amersham Biosciences, Uppsala, Suède).

*Isolement des cellules T CD4+*

Les cellules T CD4+ ont été isolées à partir des PBMCs en utilisant un protocole de sélection négative conformément aux instructions du fabricant (Miltenyi Biotec, Berlin, Allemagne). Brièvement, les PBMCs sont incubés pendant 10 minutes avec un cocktail d'anticorps biotinylés dirigés contre CD8, CD14, CD16, CD19, CD36, CD56, CD123, TCR$\gamma/\delta$ et la glycophorine A. Des billes magnétiques anti-biotine sont ensuite ajoutées pendant 15 minutes. Les cellules à éliminer sont retenues magnétiquement dans une colonne Magnetic Activated Cell Sorting (MACS®) placée dans un MACS® separator. Les cellules à isoler passent à travers la colonne, elles sont collectées et enrichies en cellules non marquées, deplétées des cellules non ciblées. L'analyse en cytométrie en flux montre que plus de 98% des cellules isolées sont des cellules CD4+.

*Isolement des lymphocytes T régulateurs*

L'isolement des lymphocytes T régulateurs humains à partir des PBMCs de donneurs adultes, a été réalisé en utilisant un kit d'isolement des lymphocytes T régulateurs CD4+CD25+ (Miltenyi Biotech, Allemagne) conformément aux instructions du fabricant. La fraction des cellules T CD4+CD25- était préservée pour les expériences de cytométrie en flux et de chimioattraction. L'analyse en cytométrie en flux montre, constamment, un enrichissement supérieur à 95% de la fraction CD4+CD25+.

**Test de prolifération cellulaire**

$1.10^5$ cellules (PBMCs ou cellules T CD4+) ont été incubées avec [methyl 3H] thymidine pendant les 18 dernières heures de culture et collectées sur un filtre en fibre de verre (Printed Filtermat A, Wallac, Turku, Finlande) en utilisant un collecteur Tomtec (Wallac). Le filtre a ensuite été scellé dans un sac après un séchage et l'addition du liquide de scintillation (Beckman Coulter, Etats-Unis). La prolifération a été mesurée après incubation en présence de [3H]thymidine (1 $\mu$Ci/puits) (PerkinElmer, Courtaboeuf, France) pour les 18 dernières heures avant la collecte. La radioactivité était mesurée en utilisant un $\beta$-compteur (1450 Trilux, Wallac, Finlande). Chaque test de prolifération a été réalisé en trois exemplaires et estimé en « count per minute » (cpm). Selon les expériences, les tests de prolifération ont été réalisés en présence de 1 $\mu$g/mL de l'isoforme courte de la Galectine 9 recombinante (Gal9S) fournie par Dr Toshiro Niki (Galpharma, Japon), de 1 $\mu$g/mL ou d'une gamme de l'anticorps anti-Galectine 9 1G3, d'un anticorps non pertinent anti-IGg1 servant de contrôle négatif (ebiosciences, Royaume Uni), de 10 $\mu$g/mL d'exosomes de C15, de 5 mM de lactose ou de sucrose (Sigma Aldrich).

**Lyse cellulaire**

La technique de mesure de lyse cellulaire est basée sur l'utilisation d'un Kit de mesure de la cytotoxicité (CytoTox-Glo Assay, Promega, USA) qui mesure une activité luciférase proportionnelle à des protéases cellulaires libérées après la cytolyse. Les tests sont réalisés en mettant en co-culture $6.10^5$ CD4+CD25+ et $2.10^5$ PBMCs autologues. Les cellules sont cultivées en plaques 96 puits à fonds ronds (Maxisorb Nunc, Danemark), dans 200 $\mu$L de milieu de culture (RPMI-1640, L-Glutamine 2 mM 1%, 0,02 mM de sodium-pyruvate, 100 U/mL de pénicilline, 100 $\mu$g/mL de streptomycine, 10% de Sérum Humain AB décomplémenté) (GIBCO BRLTM, Invitrogen®, GB). Les cellules sont activées par 1 $\mu$g/mL d'anti-CD3, préalablement coaté sur les plaques (2h à 37°C), et 100 ng/mL d'anti-CD28. Après 48h de culture, 50 $\mu$L du réactif (aminoluciférine-Glo) sont déposés dans chaque puits. Après agitation légère, les plaques de culture sont incubées pendant 15 min, à température ambiante et à l'abri de la lumière. Une première mesure de la luminescence est faite au luminomètre (Centro LB960, C Berthold Technologies, France), et elle est proportionnelle à la quantité de cellules lysées par les lymphocytes T régulateurs. Ensuite, 50 $\mu$L d'une solution de digitonine sont déposés dans chaque puits afin d'induire la lyse totale des cellules. Les plaques sont ensuite agitées puis incubées 15 min, à température ambiante et à l'obscurité, avant de faire la deuxième mesure de luminescence. Les tests sont réalisés en triplicata et les résultats sont exprimés en pourcentage de lyse.

$$Pourcentage\ de\ lyse = Viabilité\ cellulaire/\ Moyenne\ lyse\ totale\ \textbf{-}\ bruit\ de\ fond$$

$$Viabilité\ cellulaire = Moyenne\ lyse\ totale - cytolyse$$

$$Cytolyse = Moyenne\ lyse\ induite\ par\ les\ lymphocytes\ Treg\ \textbf{-}\ bruit\ de\ fond$$

**Test d'induction de l'apoptose dans les cellules Jurkat**

Les cellules Jurkat cultivées en RPMI 5% de sérum de veau fœtal sont transférées dans un milieu sans sérum (Hybridoma SFM - Life Technologies) puis incubées en présence de galectine-9 à 30 nM pendant 24h dans un puits de plaque 96 puits (100 000 cellules/puits). Le dénombrement des cellules en apoptose est fait par cytométrie en flux après marquage à l'annexine V-APC (allophycocyanine) et à l'iodure de propidium. Pour évaluer l'action protectrice des anticorps monoclonaux, la galectine est préincubée pendant 30 min en présence de l'anticorps dont la concentration finale pour l'incubation de 24h est de 10 $\mu$g/ml.

**Western Blot**

Les exosomes ont été lysés (10 min sur glace) dans du tampon PY composé de 20 mM Tris HCl, 50 mM NaCl, 5 mM EDTA, 1% Triton X 100, 0.02% azide de sodium et d'un cocktail d'inhibiteurs de protéases (Roche, Bâle, Suisse). Après centrifugation (20,000 g, 15 min, +4°C), les débris cellulaires ont été éliminés et les surnageants collectés. Les concentrations protéiques ont été mesurées en utilisant Bio Rad Protein Assay conformément aux instructions du fabricant (BioRad, Marnes la Coquette, France). Les exosomes ont ensuite été analysés par Western Blot. Brièvement, les protéines ont été séparées par une électrophorèse SDS PAGE utilisant des gels précoulés en gradient (gradient 4 12%, Bis Tris, Invitrogen) dans des conditions standards. Les protéines ont ensuite été transférées sur une membrane en nitrocellulose (Hybon dTM-C Extra, Amersham Biosciences, Royaume Uni). Cette dernière a été bloquée pendant 1 heure à température ambiente dans un tampon de blockage contenant 0.2% AuroraTM blocking reagent (MP Biomedicals, Illkirch Graffenstaden, France), 0.1% Tween20 (Sigma Aldrich) et du PBS (1X), puis elle a été incubée une nuit à 4°C avec un anticorps primaire dirigé contre la Galectine 9 : Galectin-9-CT-L1 1:100 (fourni par Galpharma, Japon). La membrane a été lavée avec un tampon de blockage, puis incubée pendant 1 heure à température ambiante avec un anticorps secondaire conjugué à la peroxydase (anti souris, 1:10000) (GE Healthcare, Wauwatosa, Etats-Unis) et lavée encore une fois avec le tampon de blockage. Les signaux spécifiques des protéines ont été visualisés grâce au Western Lightning® Plus ECL, kit amplifiant la chimiluminescence du substrat (Perkin Elmer, Boston, MA, USA) et à un analyseur d'images luminescentes LAS3000 (Fujifilm).

**Analyse FACS**

L'immunophénotypage des cellules par cytométrie en flux a été réalisé en utilisant l'appareil « FACSCalibur flow cytometer ». Après les avoir collectées, les cellules ont été lavées avec du « Phosphate-Buffered Saline » (PBS) (GIBCO-Life technologies) et marquées avec des anticorps monoclonaux conjugués à des fluorochromes (1 :10). Pour chaque essai, les isotypes contrôles (anticorps monoclonaux) appropriés ont été utilisés pour les réglages des marqueurs. Enfin, les données ont été analysées avec le logiciel FlowJo. Afin de détecter les antigènes de surface de la cellule, des anticorps de souris anti-humain ont été utilisés: CD4-phycoerythrin(PE)-cyanin(Cy)5 (BD Pharmingen, San Diego, Etats-Unis), -CD25-PE (Miltenyi Biotech, Allemagne) et -CD127-FITC (1:20) (Clinisciences, Montrouge, France) selon les instructions du fabricant.

**PCR quantitative en temps réel:**

Les ARN totaux des lymphocytes T régulateurs ont été isolés en utilisant le kit « the RNeasy minikit II » (Qiagen) selon les instructions du fabricant. La concentration et la pureté des ARN ont été mesurées par la méthode de spectrophotométrie (Ultrospec 3000, Pharmacia Biotec). L'ARN total a été stocké à -80°C jusqu'à utilisation ultérieure.

La transcription inverse des ARNm a été réalisée de la façon suivante : 2 $\mu$g de l'ARN total ont été mixés avec 5 $\mu$L du master mix composé de 1 $\mu$L oligo dT (Roche Diagnostic, Meylan, France) et de 0.1 $\mu$L RNAsin (40 U/$\mu$L, Promega, Charbonnières, France) puis incubé à 70°C pendant 5 à 10 minutes. Après 5 minutes à température ambiante, 10 $\mu$L d'un deuxième mix ont été ajoutés : 6 $\mu$L buffer 5X (Invitrogen) + 1 $\mu$L DTT 0.1 M (Invitrogen) + 2 $\mu$L dNTPs 10 mM (Amersham) + 0.1 $\mu$L RNAsin 40 U/$\mu$L (Promega) + 1 $\mu$L Superscript (Invitrogen). La réaction a été suivie d'une première incubation de 45 à 60 minutes à 45°C, une seconde incubation de 5 minutes à 95°C puis un traitement de 20 minutes avec la RNase H (Promega). Enfin, de l'eau distillée ultra-pure (GIBCO-Life Technologies) a été ajoutée afin d'obtenir une concentration finale de 10 ng d'ADN total/$\mu$L. L'ADN a été stocké à -20°C jusqu'à son utilisation ultérieure.

Les transcrits ont été quantifiés en utilisant la PCR en temps réel (RT-PCR) avec le système de détection de séquence Mx3005PTM (Agilent technologies, France), dans des plaques optiques de 96 puits (Eurogentec S. A., Belgium). Dans chaque puits, 10 $\mu$L de couple spécifique de primers, conçu pour la RT-PCR et acheté chez MWG-Biotech (Allemagne), ont été disposés à une concentration finale de 10 pg/mL et puis conservés à - 20°C. Les gènes de ménage $\beta$-actine, Glycéraldéhyde-3-Phosphate DéHydrogénase (G3PDH), ubiquitine et Hypoxanthine guanine PhosphoRibosyl Transférase (HPRT) ont été utilisés comme contrôles dans chaque plaque. Les réactions PCR ont été réalisées selon les instructions du fabricant, à un volume final de 20 $\mu$L et pour un $\mu$L d'ADNc (équivalent à 10 ng d'ARN total /$\mu$L), en utilisant du 2X MESA GREEN qPCR MasterMix Plus pour SYBR® 258 Assay (Eurogentech) contenant de la Meteor Taq DNA polymérase, MgCl2 (concentration finale de 4 mM), dNTPs (incluant dUTP), SYBR® 260 Green I, des stabilisateurs et des références passives requises pour la normalisation du signal et un tampon avec des composants optimisés

Le programme PCR incluait une dénaturation initiale et une activation de la Meteor Taq pendant 5 minutes à 95°C, suivies par 40 cycles standard d'amplification comme suit : 15 secondes à 95°C (dénaturation), 1 minute à 60°C (synthèse et élongation). Les produits fluorescents ont été détectés à la dernière étape de chaque cycle. Une analyse des courbes de fusion a été réalisée immédiatement après amplification, selon les instructions du fabricant.

Les réactions de PCR quantitative ont été utilisées pour quantifier l'expression du gène de la galectine 9 par les lymphocytes T régulateurs. Les gènes de ménage β-actin, G3PDH, ubiquitine et Hypoxanthine guanine Phospho-Ribosyl Transférase (HPRT) ont été utilisés comme contrôles. Tous les primers ont été conçus pour la RT-PCR et achetés chez MWG-Biotech (Allemagne). Une analyse quantitative a été réalisée en se basant sur la valeur « cycle threshold » (CT) ou seuil de cycle pour chaque puits et calculée en utilisant le logiciel MxPro. Chaque valeur individuelle a été normalisée en utilisant la moyenne des 4 gènes de ménage selon la méthode standard du $\Delta CT$ : $\Delta CT = C_T - C_{T\,gènes\,de\,ménage}$. Pour la comparaison entre les groupes, l'expression relative des gènes était exprimée en $2^{-\Delta\Delta C_T}$ donnant une valeur arbitraire de 1 pour l'échantillon de référence.

**Fabrication des hybridomes produisant les anticorps monoclonaux dirigés contre la galectine-9, 1G3 et 2E12.**

Une protéine recombinante représentant la portion C-terminale de la galectine 9 humaine a été utilisée comme immunogène (résidus 191 à 355 de l'isoforme longue de la galectine- 9). Elle a été produite dans E. coli sous forme d'une protéine de fusion GST. Après séparation de l'étiquette GST, la protéine a été purifiée par chromatographie d'exclusion.

Les immunisations ont été effectuées par la compagnie PX'Therapeutics (Grenoble, France). Cinq souris BALB-c femelles, âgées de huit semaines, ont été immunisées avec la portion C-terminale de la galectine 9 mentionnée ci-dessus. Pour les immunisations, 40 microgrammes de protéine ont été injectés par voie intra-péritonéale au jour 0, 22, 37 et 54 en association à l'adjuvant complet de Freund pour la première injection, ou avec l'adjuvant de Freund incomplet pour les injections suivantes. La qualité de l'immunisation a été évaluée par un test ELISA, décrit ci-dessous, sur des échantillons de sérum provenant des souris immunisées. La même préparation de galectine 9 C-terminale recombinante a été utilisée pour les immunisations d'une part et pour les tests ELISA d'autre part. Ces tests ont permis de montrer une bonne immunisation chez les cinq souris traitées.

Trois jours après le dernier rappel, les deux souris qui avaient donné la meilleure réponse ont été sacrifiées et leurs splénocytes ont été recueillis. Ces splénocytes ont été utilisés pour la fusion avec des cellules de myélome murin Sp2/O, soit en milieu liquide, soit en milieu semi-solide, avec des rapports respectifs de 5:1 et 2:1. Les surnageants d'hybridomes ont ensuite été évalués par un test ELISA, pratiqué comme le précédent et tel que décrit ci-dessous, sur la préparation de galectine 9 recombinante mentionnée ci-dessus.

La fusion semi-solide a été un succès et 39 hybridomes sécrétant des anticorps réagissant avec la galectine 9 en ELISA ont été obtenus. Sept d'entre eux ont été sélectionnés en raison d'une sécrétion d'immunoglobulines particulièrement abondante et d'une réactivité élevée en ELISA. Ces sept hybridomes ont ensuite été soumis à de nouveaux criblages fonctionnels pour étudier les propriétés neutralisantes anti-galectine 9 des anticorps produits.

Le test ELISA a été effectué de la manière suivante. La protéine recombinante représentant la portion C-terminale de la galectine 9 a été adsorbée dans les puits de plaques de microtitration 96 puits (50 ng/puits)(Greiner Bio -One, Courtaboeuf, France) de la façon suivante : mise en solution dans du tampon carbonate / bicarbonate 0,05 M à pH 9,6 et incubation dans les puits pendant 1h à température ambiante. Après lavage avec du PBS contenant 0,1% de Tween-20, les puits ont été saturés avec 3% de sérum-albumine bovine (BSA) en solution dans du PBS à température ambiante pendant 1h. Ils ont ensuite été incubés avec les sera de souris ou les surnageants d'hybridomes à tester. Sera et surnageants d'hybridomes ont été dilués dans du PBS avec 1 % de BSA puis incubés dans les puits à température ambiante pendant 2h. Après une étape de lavage avec du PBS-Tween-20 à 0,1%, les plaques ont été traitées par un anticorps secondaire marqué à la peroxydase (chèvre anti-souris). La révélation finale a lieu après addition de substrat (3,3', 5, 5' Tetramethylbenzidine ou TMB ; Thermo Fisher Scientific) et mesure de l'absorbance à 405 et 620 nm sur un lecteur de microplaques Multiskan Ex (Thermo Fisher Scientific).

**Essais *in vivo* sur des souris transgéniques**

Des souris immunodéficientes sont d'abord splénectomisées puis xenotransplantées avec une lignée cellulaire de C666-1 de CNP modifiée pour exprimer la luciférase, ce qui permet de suivre la croissance tumorale en bioluminescence par imagerie sur animal vigile. Le système immunitaire des souris est reconstitué et humanisé suite à l'injection de PBMCs humains plus ou moins enrichis en lymphocytes T régulateurs (2% de lymphocytes T régulateurs dans les PBMCs à l'origine, et ajout de 6 à 10% de lymphocytes T régulateurs dans les PBMCs). Il a déjà été montré que les PBMCs, dans une certaine mesure, sont capables de limiter la croissance de la tumeur et que l'enrichissement en lymphocytes T régulateurs n'entrave pas cet effet (Moralès et al, Activation of a Helper and Not Regulatory Human CD4+ T Cell Response by Oncolytic H-1 Parvovirus ; PLoS One. 2012;7(2):e32197). L'anticorps anti-Gal-9 1G3 a ensuite été injecté et son effet sur la croissance tumorale a été évalué.

Le protocole expérimental suivant a été suivi :
Les souris SCID âgées de 6 à 8 semaines subissent une splénectomie totale. Après 7 jours, ces mêmes souris sont xénotransplantées en sous cutanée avec des cellules d'une lignée tumorale C666-luc (exprimant la luciférase) issues d'un Carcinome du Nasopharynge (CNP). Le même jour, les souris reçoivent en intra-péritonéal, 30 à 50 millions de PBMC pour la reconstitution du système immunitaire enrichis ou non de 10% en lymphocytes T régulateurs et, selon les animaux, 2, 20 ou 200μg d'anticorps IgG1 ou d'anticorps 1G3 en sous cutané selon le schéma suivant (3 souris/groupe) :

- **Gp1** : IgG1 isotype (non reconstituées)
- **Gp2** : 1G3 (non reconstituées)
- **Gp3** : reconstituées +10% Treg + IgG1 isotype
- **Gp4** : reconstituées (non traitées)
- **Gp5** : reconstituées +10% Treg + 1G3

Toutes les souris reçoivent 3 μg de CPG-ODN2216 (commercialisé par MILTENYI BIOTECH) afin d'activer la réponse immune.

Le suivi est effectué trois fois par semaine pendant 4 semaines par des lectures de luminescence sur la plateforme d'imagerie du petit animal (IVIS® Lumina XRMS, PerkinElmer) et par des mesures manuelles du volume et de la masse tumorale. Aux jours 7, 14 et 21 post-transplantation, les souris reçoivent un rappel de CPG-ODN2216 (commercialisé par MILTENYI BIOTECH) et, en fonction des groupes présentées précédemment, un rappel d'anticorps 1G3 ou IgG1. Avant le premier rappel, un échantillon de sang est prélevé afin de vérifier la reconstitution par cytométrie de flux (cf protocole page 58). Après 28 jours de mesure, les souris sont sacrifiées, les tumeurs récupérées et cryopréservées en prévision de la préparation de lames d'Immuno-Histochimie ou Immunofluorescence et un échantillon de sang est de nouveau prélevé pour analyse par cytométrie de flux.

L'impact de différentes concentrations d'1G3 et d'isotype IgG1 [2, 20 et 200μg/mL] a été évalué sur (i) le poids des souris, (ii) le volume tumoral et (iii) la masse tumorale au sacrifice.

(i) <u>Analyse de l'impact de l'injection de l'anticorps 1G3 sur le poids des souris</u>

Les souris entrant sont régulièrement pesées [du jour 5 au Jour 28].

(ii) <u>Analyse de l'impact de 1G3 sur le volume tumoral (mesure manuelle)</u>

Plusieurs expériences individuelles ont été effectuées sur des souris SCID humanisées suivant le protocole décrit ci-dessus et en faisant varier la concentration de 1G3 et de 1G1 injectés. Le volume des tumeurs a été mesuré manuellement.

Par ailleurs, afin d'obtenir un graphique représentatif de l'impact du 1G3 sur le volume tumoral, des résultats moyennés regroupant 6 expériences in vivo indépendantes ont été réalisés avec l'anticorps 1G3 ou l'isotype contrôle (20 μg).

L'analyse statistique a été réalisée via un test Rank Sum Mann Whitney (*p<=0.05, **p<=0.001).

Le volume de la tumeur a également été mesuré par analyse de la bioluminescence émise par les cellules tumorales qui expriment le gène de la luciférase, après injection de luciférine. La luminescence est mesurée via l'utilisation du système de mesure de bioluminescence de la souris (IVIS LUMINA). Six expériences individuelles ont été réalisées sur les souris SCID humanisées suivant le protocole décrit ci-dessus et en faisant varier la concentration de 1G3 et de 1G1 injectés.

Par ailleurs, afin d'obtenir un graphique représentatif de l'impact du 1G3 sur le volume tumoral, des résultats moyennés de 6 expériences in vivo indépendantes ont été réalisés avec l'anticorps 1G3 ou l'isotype contrôle (20 μg).

Enfin, des tumeurs de souris traitées avec 1G3 ou l'isotype contrôle IgG1 ont été photographiées sur papier millimétré pour mesurer la taille de la tumeur au moment de l'euthanasie.

(iii) <u>Analyse de l'impact de 1G3 sur la masse de la tumeur</u>

Des tumeurs de souris traitées avec 1G3 ou l'isotype contrôle IgG1 ont été pesées après sacrifice. La masse est exprimée en grammes.

L'analyse statistique a été réalisée par un test de Rank Sum Mann Whitney (*p<=0.05, **p<=0.001) (Fig. 22).

**Analyse de la capacité de 1G3 à inhiber l'induction de lymphocytes T régulateurs par la galectine 9**

Il a été montré que la galectine 9 est capable d'induire la différentiation des cellules T CD4 naïves en lymphocytes T regs (Seki, Oomizu et al. 2008), ce qui renforce l'importance de cette lectine dans les phénomènes d'exhaustion de la réponse immune anti-tumorale.

Tout d'abord, il a donc été vérifié si la galectine 9 peut transformer ou non des cellules T conv en T regs. Des cellules T conv ont été cultivées pendant 3 ou 5 jours en conditions d'activation ou non-activation, avec ou sans un isoforme de la galectine 9 (Gal-9 S ou Gal-9 M), avec ou sans 1G3. Après ces phases de conditionnement, les cellules sont récupérées, lavées et analysées par cytométrie de flux et mises en co-culture avec les PBMCs oudes T conv autologues. Le milieu de culture a également été récupéré pour une analyse ELISA.

Le protocole est le suivant : les PBMC ont été isolés par purification sur gradient de Ficoll et les Lymphocytes T conventionnels ont été isolées sur des colonnes magnétiques selon le protocole du fournisseur (Miltenyii Biotech, T Cell Isolation kit). Une partie des PBMC est conservée en condition de non-activation et les Tconv autologues sont cultivées pendant 5 jours en condition d'activation ou de non-activation. L'activation se fait par l'anti CD3 ($1\mu$g/mL) (fourni par l'équipe d'Anne Tsicopoulos, CIIL) préalablement coaté sur les plaques (2H à 37°C) et l'anti CD28 (100ng/mL) (Clinisciences, France).

Ces cellules T sont conditionnées ou non par les isoformes de la Gal-9 S ou M à $2\mu$g/mL en présence ou non de l'anticorps 1G3 à $3\mu$g/mL. Au bout des 5 jours, les Tconv conditionnés sont lavés, la viabilité est établie par comptage au bleu Trypan* (cf ci-dessous), le surnageant est récupéré en vue de tests ELISA et $10^5$ cellules sont analysées par cytométrie de flux afin de mesurer l'expression des marqueurs CD4, CD25 et CD127. Le reste des cellules conventionnelles est mis en contact avec les PBMC autologues qui avaient été conservés pour le test de MLR.

En effet, les tests de suppression sont réalisés par MLR (Mixed lymphocytes réaction) en mettant en co-culture, $10^5$ Tconv conditionnés avec $10^5$ PBMCs autologues (ration 1 : 1), en présence ou non de l'anticorps 1G3 ou 2E12. Les cellules sont activées avec $1\mu$g/mL d'anti CD3, préalablement coaté sur les plaques (2H à 37°C) et l'anti CD28 (100ng/mL).

Après 3 jours de co-culture, la prolifération est évaluée par incorporation de $1\mu$Ci/puits de thymidine tritiée ($^3$H Th) (GE HEALTHCARE, France), 18H avant la fin de la culture. Après 3 jours, les plaques sont filtrées sur filtres en fibres de verres (Perkin Elmer, France). Le filtre est incubé dans un liquide un liquide de scintillation (Beckman Instruments Inc, Ready safe, USA) et lu au compteur de scintillation (1450 Trilux, Wallac, Finlande). Les résultats sont enfin exprimés en coup par minute (cpm).L'analyse statistique a été réalisé par un test de Rank Sum Mann Whitney (*p<=0.05, **p<=0.001)

## Comptage au Bleu trypan

Le colorant azoïque vital (Bleu Trypan) pour colorer les cellules mortes. Le comptage est évaluée par incorporation de 1 volume de solution de bleu trypan 0.4% (Sigma, USA) pour un volume de suspension cellulaire. Après 3min, le mélange est déposé sur une lame de Thomas. Les cellules mortes qui apparaissent en bleu et les cellules vivantes, réfringentes, sont comptées. Les mesures sont réalisées en triplicata et les résultats exprimés en pourcentage de cellules vivantes.

## Mesure de la sécrétion de la Galectine 9 par ELISA

50 $\mu$L d'une solution à 0,2$\mu$g/mL d'anticorps anti-Gal9 (Clone : SEA309Hu- Uscn Life Science Inc, USA) sont fixées dans des plaques 96 puits (NUNC, Danemark), pendant 1 nuit à 4°C. Après 4 lavages au PBS 1X (Euromedex, France)-Tween (Sigma Aldrich, USA) 0,05%, les plaques sont saturées avec du PBS-BSA 3% (Sigma-Aldrich®, USA) pendant 2H à température ambiante. Puis, elles sont lavées 3 fois avec du PBS-Tween 0,05%. 100 $\mu$L/puits de surnageant de culture des Treg activés ou non sont déposés en duplicate, et incubés pendant 2h à température ambiante. Les Treg sont classiquement activés par l'anti CD3 ($1\mu$g/mL) (fourni par l'équipe d'Anne Tsicopoulos, CIIL) préalablement coaté sur les plaques (2H à 37°C) et l'anti CD28 (100ng/mL) (Clinisciences, France).

Sur chaque plaque, une gamme de Galectine 9 recombinante (Uscn Life Science Inc, USA) d'une concentration de 2,5ng/mL à 2,5pg/mL, dans du PBS-BSA 1% est faite.

Après 3 lavages au PBS-Tween, 100$\mu$L/puits d'anticorps secondaire biotinylé, à 1$\mu$g/mL, sont incubés pendant 1H à température ambiante. La plaque est à nouveau lavée 3 fois et la réaction est amplifiée par l'ajout de 100$\mu$L/puits de streptavidine-peroxydase au 1/10000, pendant 30 minutes à température ambiante. Après 4 lavages, les plaques sont révélées par 100$\mu$L/puits de solution de révélation à l'OPD (O-Phenylenediamine dihydrochloride) (Sigma-Aldrich®, USA) à 1 mg/mL pendant 10 minutes à l'obscurité. Cette réaction est stoppée par l'ajout de 50$\mu$L/puits d'HCl (VWR, USA) 2N.

Les plaques sont ensuite lues au spectrophotomètre, à une longueur d'onde de 492nm (Multiskan Ex, ThermoLabsystems, France).

## Mesure de la viabilité des PBMC

Les tests de viabilité ont été effectués directement par la méthode CellTiter-Glo qui permet de mesurer le métabolisme mitochondrial. Le même protocole est utilisé pour évaluer la viabilité des Tconv.

$2.10^5$ de PBMCs autologues sont déposés en co-culture. Les cellules sont activées [activation par l'anti CD3 (1μg/mL) (fourni par l'équipe d'Anne Tsicopoulos, CIIL) préalablement coaté sur les plaques (2H à 37°C) et l'anti CD28 (100ng/mL) (Clinisciences, France)] ou pas selon les conditions et elles sont incubées avec l'anticorps 1G3 ou l'isotype contrôle 1G1 à la concentration de 3, 6 ou 12μg/ml. La culture se fait en plaques 96 puits à fond plat à « murs » opaques (Corning3610, Corning Incorporated, USA) dans 100μL de milieu de culture (DMEM + 4.5g/L glucose +L-glutamine, 100U/mM de pénicilline, 100μg/mL de streptomycine, 10% de sérum Humain AB décomplémenté) (GibcoBRLTM, life technologies, GB).

Le test utilise le Kit Promega CellTiter-Glo luminescent Cell Viability Assay (Promega coorporation, USA), qui utilise l'activité de la luciférase, en présence d'oxygène, pour mesurer le métabolisme cellulaire (ATP) comme indicateur de la viabilité cellulaire. Après 48H de culture, 100μL du réactif (Luciférine, luciférase et tampon contenant du magnésium) sont ajoutés dans chaque puits ; agitation de la plaque pendant 2min puis incubation pendant 15min, à température ambiante, à l'abri de la lumière. Les tests sont réalisés en triplicata et la lecture des plaques est effectuée au luminomètre (Centro LB960, C Berthold Technologies, France). Lecture 1sec/puits.

**Marquages membranaires des Tconv par Cytométrie en flux (Facs)**

Anticorps utilisés (Ac)

Anticorps primaires monoclonaux anti-humains couplés à des fluorochromes, anti-CD4-PE (Phycoérythrine) -C (Cyanine) 5 (BD PharmingenTM, USA), anti-CD25-PE (Miltenyi Biotec, France), anti-CD127- FITC (Fluorescein IsoThioCyanate) (Clinisciences, France).

Pour la compensation, l'isotype contrôle des différents anticorps monoclonaux a été utilisé.

Protocole de marquage direct

Les cellules ($2.10^5$) sont reprises dans un volume de 100μL de PBS stérile (GIBCO BRLTM, Invitrogen®, GB) et incubées pendant 30min à température ambiante et à l'obscurité avec 10μL d'anti-CD4-PC, 10μL d'anti-CD25-PE et 4μL d'anti-CD127-FITC. Les cellules marquées sont ensuite reprises avec 400μL de PBS et la fluorescence est analysée par cytométrie de flux au Facscalibur (FACS Flow Supply System, Becton Dickinson, USA).

Les résultats de cytométrie sont analysés par le logiciel Flow Jo (Tree Star Incorporation, USA). Pour certaines expériences, après l'ajout des anticorps marqués, les cellules sont fixées par ajout de 100μL de PFA 4% (Sigma Aldrich, USA) pendant 10min puis elles sont reprises dans 200μL de PBS avant l'analyse au cytomètre.

**Prolifération et Suppression (MLR) (2E12)**

Contrôle de Prolifération

Les tests de prolifération sont réalisés sur $10^5$ PBMC cultivés pendant 48H. Les cellules sont cultivées en plaques 96 puits à fonds ronds (Nunc, Danemark) dans 200μL de milieu de culture (RPMI-1640, L-glutamine 2mM 1%, 0.02mMde sodium pyruvate, 100U/mM de pénicilline, 100μg/mL de streptomycine, 10% de sérum Humain AB décomplémenté) (GibcoBRL™, Invitrogen, GB). Elles sont activées par l'anti CD3 (1μg/mL) (fourni par l'équipe d'Anne Tsicopoulos, CIIL) préalablement coaté sur les plaques (2H à 37°C) et l'anti CD28 (100ng/mL) (Clinisciences, France), en présence ou non d'anticorps 1G3 ou 2E12.

Suppression

Les tests de suppression sont réalisés par MLR (Mixed lymphocytes réaction) en mettant en co-culture, $6.10^4$ Treg avec $10^5$ PBMCs autologues, en présence ou non de l'anticorps 1G3 ou 2E12. Les cellules sont activées avec 1μg/mL d'anti CD3, préalablement coaté sur les plaques (2H à 37°C) et l'anti CD28 (100ng/mL).

La prolifération est évaluée par incorporation de 1uCi/puits de thymidine tritiée ($^3$H Th) (GE HEALTHCARE, France), 18H avant la fin de la culture. Après 48H, les plaques sont filtrées sur filtres en fibres de verres (Perkin Elmer, France). Le filtre est incubé dans un liquide un liquide de scintillation (Beckman Instruments Inc, Ready safe, USA) et lu au compteur de scintillation (1450 Trilux, Wallac, Finlande). Les résultats sont enfin exprimés en coup par minute (cpm).

## Résultats

**Analyse phénotypique des PBMCs et des lymphocytes T régulateurs**

L'analyse en cytométrie de flux (FACS) des PBMCs montre que les lymphocytes T régulateurs CD4+CD25+CD127- représentent 1% des PBMCs totaux (résultats non montrés).

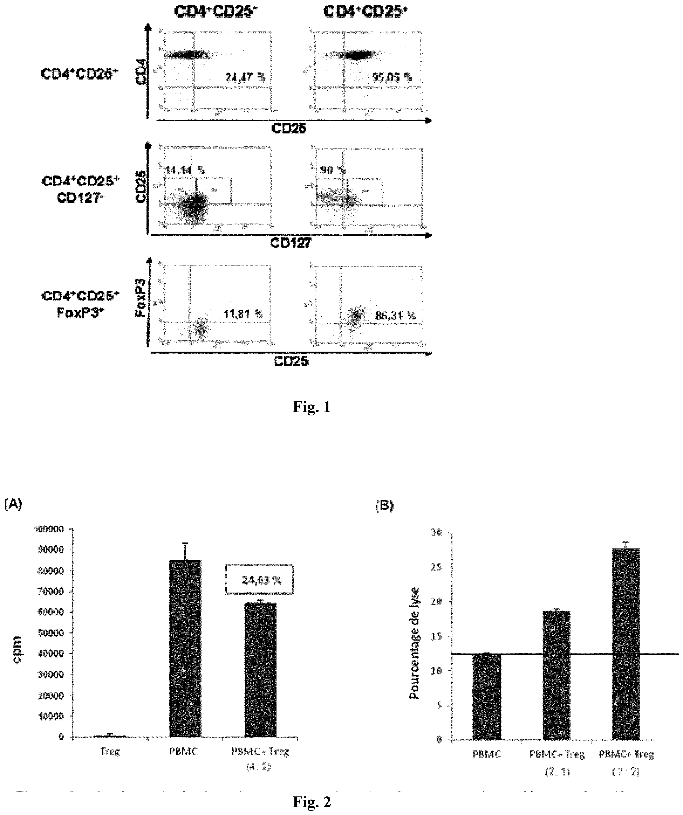

Par ailleurs, l'analyse FACS des marqueurs phénotypiques des lymphocytes T régulateurs autologues isolés ex vivo indique que 95% des lymphocytes T régulateurs sont CD4+CD25+ et que parmi ces cellules, 90% sont CD127- ou CD127low et plus de 86% FoxP3+ (Figure 1).

**Les lymphocytes T régulateurs activés ont une activité suppressive**

L'activité suppressive des lymphocytes T régulateurs humains, isolés *ex vivo* à partir du sang de donneurs sains, a été caractérisée par deux analyse fonctionnelles complémentaires: un test de suppression de la prolifération des PBMCs activés par les lymphocytes T régulateurs autologues et un test de cytolyse des PBMCs activés par les lymphocytes T régulateurs autologues (Figure 2).

La figure 2A montre que les PBMCs activés seuls prolifèrent bien in vitro tandis que les lymphocytes T régulateurs isolés ex vivo sont anergiques, même après leur activation. Cependant, la prolifération des PBMCs activés diminue de plus de 24% en présence de lymphocytes T régulateurs autologues activés pour un ratio de 4:2 (cf. Fig. 2A). Le test de prolifération (MLR) met donc bien en évidence que les lymphocytes T régulateurs isolés ex *vivo* et en condition d'activation possèdent une activité immunosuppressive.

Les résultats obtenus par le test de prolifération sont confortés par les résultats du test de cytolyse. Il est en effet mis en évidence à la figure 2B que plus le ratio PBMC : lymphocytes T régulateurs est faible, plus le pourcentage de lyse des PBMCs activés est élevé. Les lymphocytes T régulateurs induisent donc la lyse des PBMCs autologues, à différents ratios et de façon dose-dépendante.

**La galectine 9 est présente sur, et exprimée par, les lymphocytes T régulateurs**

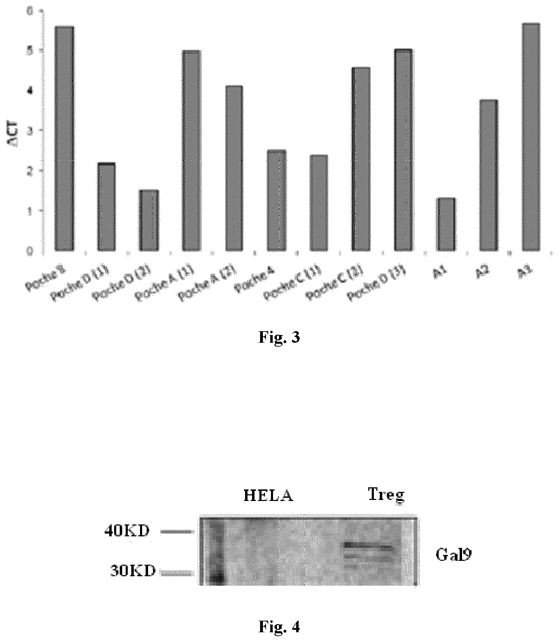

Les analyses par PCR quantitative en temps réel (Figure 3) et par Western-Blot (Figure 4) montrent que la galectine 9 est présente sur les lymphocytes T régulateurs humains isolés *ex vivo* et que ces derniers expriment la galectine 9, suggérant que les lymphocytes T régulateurs utilisent la voie de la galectine 9 pour inhiber la prolifération des lymphocytes T effecteurs.

**L'expression de la galectine 9 par les lymphocytes T effecteurs diminue lors de l'activation ; l'expression de la galectine 9 par les lymphocytes T régulateurs augmente lors de l'activation**

La figure 14 met en évidence qu'alors que les lymphocytes T conv et les lymphocytes T régulateurs non activés produisent des quantités très faibles de galectine 9 (<10pg/ml : soit en dessous du seuil de détection d'un test ELISA) les lymphocytes T régulateurs humains activés sont capables de synthétiser et de sécréter de la galectine 9 dans le milieu extra-cellulaire. Par ailleurs, cette sécrétion de la galectine 9 est significativement plus importante lorsque les lymphocytes T régulateurs sont activés, reliant cette sécrétion à leur fonction suppressive.

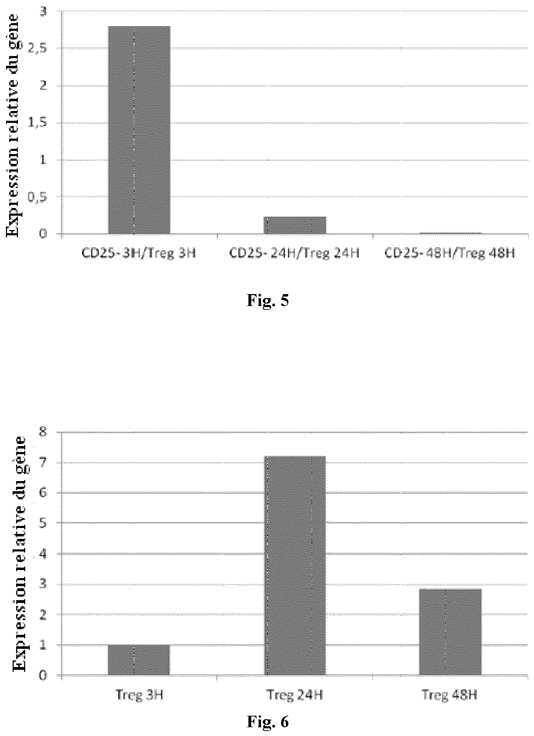

La figure 5 met en évidence que le rapport lymphocytes T CD4+ conventionnels : lymphocytes T régulateurs diminue considérablement pendant l'activation.

Les lymphocytes T CD4+ conventionnels activés expriment peu le gène codant la galectine 9 et cette expression diminue significativement au cours de leur activation.

En revanche, l'expression du gène codant la galectine 9 par les lymphocytes T régulateurs augmente au cours de leur activation (cf. figures 5 et 6)

Ainsi, les lymphocytes T effecteurs constitutivement activés, ayant une action anti-tumorale, ne seront pas la cible de l'anticorps anti-gal9. En revanche, les lymphocytes T régulateurs activés et donc fonctionnels, seront une cible privilégiée de l'anticorps anti-gal9.

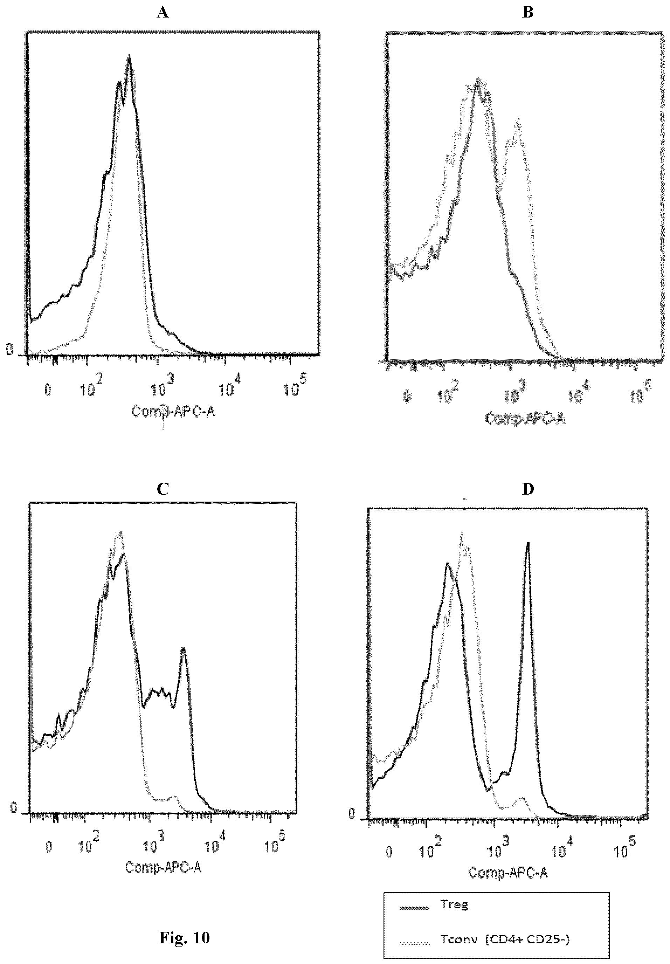

L'analyse de l'expression protéique différentielle de la galectine 9 dans les lymphocytes T régulateurs et les cellules T CD4+, a été confortée par cytométrie de flux. Comme visible à la figure 10, l'expression basale est faible et quasiment identique entre les lymphocytes T régulateurs et les cellules T CD4+ fraichement isolés. Néanmoins, il est constaté qu'après activation du TCR (anti-CD3/anti-CD28), le profil d'expression change avec l'apparition d'une population dans les lymphocytes T régulateurs qui exprime très fortement la galectine 9. Cette surexpression persiste et croit proportionnellement au temps alors que l'expression basale de la galectine 9 reste faible dans les cellules T CD4+ conv, même après l'activation (Fig 10).

Il est ainsi démontré que la surexpression de la galectine 9 est spécifique des lymphocytes T régulateurs activés et qu'il y a une expression très faible de la galectine 9 dans les cellules T CD4+ conv en conditions basales et après activation. Le risque de cibler les lymphocytes T CD4+ effecteurs est ainsi écarté, ce qui permet au patient soigné selon l'invention de maintenir ses défenses immunitaires.

**L'activité suppressive des lymphocytes T régulateurs est inhibée par l'anticorps anti-galectine 9 1G3**

Pour évaluer in vitro l'impact de l'anticorps 1G3 sur l'activité des lymphocytes T régulateurs, un test de prolifération cellulaire basé sur l'incorporation de la thymidine tritiée a été utilisé.

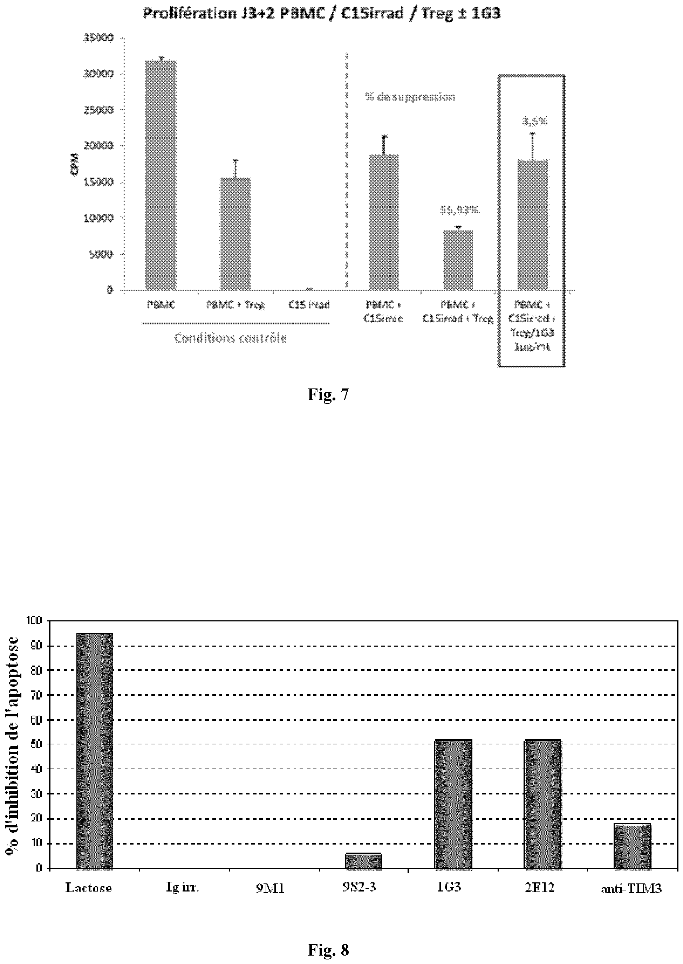

La figure 7 présente les résultats du test de prolifération des PBMCs en présence des C15 irradiées en présence ou non de lymphocytes T régulateurs et en présence ou non d'anticorps 1G3 à la concentration de 1μg/mL.

Premièrement, il est confirmé par les contrôles positifs du test que l'activation des PBMCs entraine bien une augmentation de leur prolifération et que la présence des lymphocytes T régulateurs induit bien une diminution de la prolifération cellulaire des PBMCs.

Il est également mis en évidence que l'irradiation des cellules tumorales C15 induit bien un arrêt de leur prolifération. Les cellules C15 sont anergiques.

La présence des cellules tumorales C15 induit bien une diminution de la prolifération cellulaire des PBMCs humains.

Il est ensuite mis en évidence que la présence des lymphocytes T régulateurs dans la co-culture de PBMCs et de C15 induit une diminution supplémentaire significative de la prolifération, d'environ 56%.

La figure 7 montre clairement que, de façon inattendue, la présence de l'anticorps 1G3 permet de restaurer la prolifération des PBMCs. Il est ainsi suggéré que l'anticorps 1G3 neutralise la galectine 9 présente sur, et exprimée par, les lymphocytes T régulateurs. Par conséquent, il est mis en avant que l'anticorps 1G3 inhibe l'activité suppressive des lymphocytes T régulateurs.

**L'anticorps anti-galectine 9 1G3 a une efficacité supérieure à d'autres anticorps anti-galectine 9**

Pour comparer l'effet de l'anticorps 1G3 à d'autres anticorps anti-galectine 9, voire à un anticorps anti-Tim 3, sur l'inhibition de l'activité suppressive des lymphocytes T régulateurs, plusieurs tests ont été effectués.

Ainsi, l'effet de différents anticorps anti-galectine 9 sur l'inhibition de l'apoptose induite par la galectine 9, ainsi que l'effet d'anticorps anti-galectine 9 sur la restauration de la prolifération après traitement de PBMCs humains avec de la galectine 9 ont été analysés. Les anticorps anti-galectine 9 testés en comparaison de 1G3 ne reconnaissent pas le même épitope de la galectine 9 que l'anticorps 1G3.

Un anticorps anti-Tim 3 a également été testé. En effet, certaines théories ont été soulevées concernant un lien entre le récepteur Tim-3, qui serait présent sur les lymphocytes T, et l'effet pro-apoptique de la galectine 9.

Inhibition de l'apoptose induite par la galectine 9

La figure 8 présente les résultats du test sur l'effet des anticorps testés sur l'apoptose des Jurkat induite par la galectine 9 recombinante.

Les anticorps testés correspondent à l'anticorps 9M1 (anti-galectine 9), 9S2-3 (anti-galectine 9), 1G3 (anti-galectine 9), un anticorps anti-TIM3, 2E12 (anti-galectine 9).

Comme visible sur la figure 8, la protection contre l'apoptose des Jurkat est meilleure avec l'anticorps 1G3 qu'avec les autres anticorps anti-galectine 9 9S2-3 et 9M1 ou l'anticorps anti-TIM3.

Restauration de la prolifération après traitement avec de la galectine 9

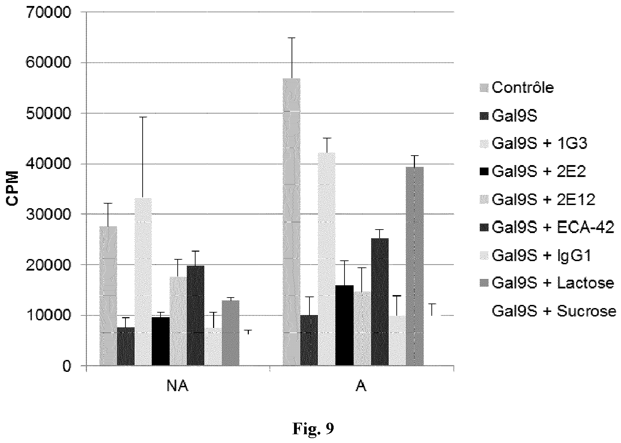

La figure 9 présente les résultats du test sur l'effet des anticorps testés sur la prolifération de PBMCs humaines traités au préalable avec de la galectine 9.

Les anticorps testés correspondent à l'anticorps ECA-42 (anti-galectine 9), 1G3 (anti-galectine 9), 2E2 (anti-TIM3) et 2E12 (anti-galectine 9).

Comme visible sur la figure 9, la prolifération des PBMCS humains est restaurée de manière plus efficace avec l'anticorps 1G3 qu'avec les autres anticorps anti-galectine 9 ECA-42 et 2E12 ou l'anticorps anti-TIM3 (2E2).

Effet sur les lymphocytes T conv (T CD4+)

La figure 11 présente les résultats du test sur l'effet de la galectine 9 et du 1G3 sur les lymphocytes T conv (T CD4+) par mesure de la prolifération sur cellules fraichement isolées, via l'incorporation de thymidine tritiée durant les 18 dernières heures de culture. Les résultats sont également donnés en coups par minute (CPM).

Tout comme les PBMCs (figure 9), les Tconv isolés ex-vivo prolifèrent en condition d'activation. Il est également mis en évidence que la galectine 9 inhibe significativement la prolifération des Tconv, de la même manière que les PBMCs.

Il est enfin à noter sur cette figure 11 que l'anticorps anti-galectine 9 (1G3) n'a pas d'effet sur la prolifération des Tconv.

Analyse de l'effet cytotoxique de l'anticorps 1G3 et de son isotype contrôle sur la prolifération des PBMCs

L'effet de doses croissants d'anticorps 1G3 ou de son isotype contrôle (IgG1) a été analysé sur les PBMCs en mesurant la prolifération sur cellules fraichement isolées extraites du sang de 3 donneurs par incorporation de thymidine tritiée durant les 18 dernières heures de la culture. Les résultats ont été obtenus en CPM.

Il est observé à la figure 12 que les PBMCs isolés ex-vivo prolifèrent in-vitro en condition d'activation. Il est par ailleurs à noter que l'anticorps 1G3 et son isotype contrôle IgG1 n'affectent pas la prolifération des PBMCs, même avec des doses importantes d'anticorps monoclonal (de 3 à 12 μg/ml).

Analyse de l'effet cytotoxique de l'anticorps 1G3 et de son isotype contrôle sur la viabilité des PBMCs

L'augmentation de la dose de mAb 1G3 et de son isotype contrôle (1G1) ont été analysées en mesurant la viabilité de PBMCs fraîchement isolés à partir de 3 donneurs par analyse luminométrique. Le métabolisme mitochondrial a été mesuré pendant 5 jours d'activation. Les résultats ont été obtenus en RLU.

La figure 13 montre que les PBMCs isolés en ex vivo maintiennent et augmentent leur viabilité *in vitro* dans des conditions d'activation. Cela démontre également que l'1G3 et l'isotype 1G1 ne modifient pas la viabilité des PBMCs, même à forte dose d'anticorps (de 3 à 12 μg/ml).

Il a ainsi été démontré que l'anticorps 1G3 n'affecte ni la prolifération, ni la viabilité des PBMCs et des T CD4+ humains. Ainsi, le risque d'effet secondaire, tel que l'induction d'une immunosuppression qui serait favorable à la progression tumorale, est réduit. Cela permet un meilleur maintien des défenses immunitaires, notamment anti-tumorales, chez le patient soigné selon l'invention.

Analyse de l'effet neutralisant de 1G3 sur l'activité suppressive des lymphocytes T régulateurs

Le potentiel d'inhibition de la fonction des lymphocytes T régulateurs par l'anticorps 1G3 a été analysé par des essais de prolifération d'une réaction leucocytaire mixte de lymphocytes T régulateurs et T conventionnels.

Comme attendu, les résultats ont démontré que les lymphocytes T régulateurs inhibent la prolifération de ces cellules en co-culture (figure 15). De plus, une pré-culture de 2 heures avec l'1G3 a suffi pour inverser cet effet inhibiteur. L'utilisation de l'isotype contrôle d'1G3, qui n'a aucun effet sur les Tregs, permet de conclure que cette inversion de l'inhibition induite des lymphocytes T régulateurs est spécifique et passe via la galectine 9.

Afin de déterminer si 1G3 inhibe les lymphocytes T régulateurs en agissant sur la galectine 9 soluble ou directement sur les lymphocytes T régulateurs, un test a été effectué avec un inhibiteur bloquant la galectine 9 soluble, et comparé avec les résultats obtenus avec l'anticorps 1G3.

Les résultats montrent clairement que l'inhibiteur chimique n'a eu aucun effet significatif sur l'activité suppressive des lymphocytes T régulateurs. L'utilisation avec l'anticorps 1G3 a, à nouveau, provoqué une inversion de la suppression induite par les lymphocytes T régulateurs. Ces résultats confirment ainsi que la galectine 9 intervient dans la suppression induite par les lymphocytes T régulateurs et que 1G3 est capable d'inverser cette inhibition.

**Neutralisation *in vivo* de l'activité suppressive des lymphocytes T régulateurs**

L'impact de différentes concentrations d'1G3 et d'isotype IgG1 [2, 20 et 200μg/mL] a été évalué sur (i) le poids des souris, (ii) le volume tumoral et (iii) la masse tumorale au sacrifice.

(i) Analyse de l'impact de l'injection de l'anticorps 1G3 sur le poids des souris

Comme visible à la figure 18 A et B, les souris traitées au 1G3 sont plus légères comparativement à celles traitées par l'isotope contrôle. Ces résultats suggèrent que la réduction de poids de la souris traitée peut être liée à la réduction de la tumeur.

(ii) Analyse de l'impact de 1G3 sur le volume tumoral (mesure manuelle)

Les résultats obtenus représentés à la figure 19 A-D indiquent un effet positif du 1G3, qui induit une limitation significative de la croissance tumorale comparativement à l'isotype contrôle.

Par ailleurs, les résultats présentés à la figure 20 indiquent clairement une différence significative entre une immunisation par 1G3 ou son isotype contrôle, la croissance tumorale par 1G3 étant largement limitée.

Ces résultats sont également confirmés par les mesures par bioluminescence (Figure 21 A-D, Figure 22), 1G3 induisant une limitation de la croissance tumorale comparativement à son isotype contrôle.

Enfin, les photographies des tumeurs (non représentées) mettent aussi en évidence l'effet positif de 1G3 qui induisant une limitation de la croissance tumorale comparativement à son isotype contrôle.

(iii) Analyse de l'impact de 1G3 sur la taille de la tumeur

Les résultats concernant la masse de la tumeur (figure 23) confirment, à leur tour, l'effet bénéfique de 1G3 par rapport à son isotype contrôle.

**Analyse de la capacité du 1G3 à inhiber l'induction de lymphocytes T régulateurss par la galectine 9**

Il a été montré que la galectine 9 est capable d'induire la différentiation des T CD4 naïves en Tregs (Seki et al, Galectin-9 suppresses the generation of Th17, promotes the induction of regulatory T cells, and regulates experimental autoimmune arthritis ; Clin Immunol. 2008 Apr;127(1):78-88. doi: 10.1016/j.clim.2008.01.006. Epub 2008 Feb 20), ce qui renforce l'importance de cette lectine dans les phénomènes d'exhaustion de la réponse immune anti-tumorale.

Les résultats présentés à la figure 25 indiquent clairement que les T CD4+ conv conditionnés avec chaque isoforme de la galectine 9 acquièrent un phénotype suppressif et inhibent la prolifération des PBMCs. Cette inhibition est plus forte avec la forme S (courte) de la galectine 9 qu'avec la forme M (moyenne). Il a également été constaté que l'ajout d'1G3 lors du conditionnement inhibe l'établissement de ce phénotype suppressif et restaure la prolifération de manière significative en neutralisant la galectine 9.

Par ailleurs, comme montré à la figure 24, les analyses FACS des cellules à la fin de l'essai indiquent que chaque isoforme de la galectine 9 engendre une augmentation fr l'expression du CD25. Or, comme le montre la figure 24, les cellules activées surexpriment en temps normal le CD25.

Les résultats indiquent clairement que l'addition sur les Tconv de galectine 9 (i) engendre une légère augmentation de l'expression du CD4 avec la forme S ou la forme M de la galectine 9, comparativement au T conv activés; (ii) n'a pas d'effet sur l'expression du CD127 que ce soit pendant la phase de conditionnement avec la forme M ou S de la galectine 9; (iii) entraîne une augmentation importante de l'expression du CD25 avec la forme M et la forme S de la galectine 9 par rapport aux Tconv activés.

La figure 24 indique donc que la pré-incubation des Tconv avec la forme M ou S de la Galectine 9 augmente l'expression des marqueurs CD4 et CD25 en faveur d'un phénotype possiblement plus suppresseur. Néanmoins, cette hypothèse n'est recevable que si elle est corrélée à une activité suppressive de ces Tconv conditionnés, permettant de valider le phénotype suppressif, ce qui est le cas (cf figure 25).

Pour conclure, il est mis en évidence que la Galectine 9 induit une conversion des lymphocytes T CD4+ conventionnels en T CD4+ immunosuppresseurs. L'anticorps 1G3 est capable de neutraliser l'induction de cette conversion et d'ainsi favoriser le maintien d'une réponse immunitaire anti-tumorale chez le patient soigné selon l'invention.

**Analyse de l'effet neutralisant de 2E12 sur l'activité suppressive des Tregs**

Le potentiel d'inhibition de la fonction suppressive des lymphocytes T régulateurs par l'anticorps 2E12 a été analysé par des essais de prolifération d'une réaction leucocytaire mixte de lymphocytes T régulateurs avec des PBMCs autologues (2 donneurs indépendants).

Les résultats, visibles à la figure 26, mettent en évidence qu'une pré-culture de 2 heures des lymphocytes T régulateurs avec le 2E12 a suffi pour inverser l'effet inhibiteur de la prolifération des cellules par les lymphocytes T régulateurs. L'utilisation de l'isotype contrôle du 2E12, qui n'a aucun effet sur les lymphocytes T régulateurs, nous permet à nouveau de conclure que cette inversion de l'inhibition induite des lymphocytes T régulateurs est spécifique et passe via la galectine 9.

Par ailleurs, il est observé que l'anticorps 2E12, tout comme 1G3, est capable d'inverser cette inhibition. L'efficacité de 2E12 est sensiblement moins élevée que le 1G3 cependant, en ce qu'une légère diminution de la prolifération des PBMC est observée, contrairement à 1G3.

**REFERENCES**

(1) Grossman WJ, Verbsky JW, Barchet W, Colonna M, Atkinson JP, Ley TJ. « Human T regulatory cells can use the perforin pathway to cause autologous target cell death". Immunity. 2004 Oct; 21(4):589-601.

(2) Garin MI, Chu CC, Golshayan D, Cernuda-Morollón E, Wait R, Lechler RI. "Galectin-1: a key effector of regulation mediated by CD4+CD25+ T cells". Blood. 2007 Mar 1;109(5):2058-65

(3) Johnson BD, Jing W, Orentas RJ. « CD25+ regulatory T cell inhibition enhances vaccine-induced immunity to neuroblastoma". J Immunother. 2007 Feb-Mar;30(2):203-14.

(4) McHugh RS, Whitters MJ, Piccirillo CA, Young DA, Shevach EM, Collins M, Byrne MC. "CD4(+)CD25(+) immunoregulatory T cells: gene expression analysis reveals a functional role for the glucocorticoid-induced TNF receptor". Immunity. 2002 Feb;16(2):311-23.

(5) Wing K, Onishi Y, Prieto-Martin P, Yamaguchi T, Miyara M, Fehervari Z, Nomura T, Sakaguchi S. "CTLA-4 control over Foxp3+ regulatory T cell function". Science. 2008 Oct 10;322(5899):271-5. (5) Zahran AM et al, Int J Clin Oncol. 2013 Sep 26.

(6) Fisson S1, Darrasse-Jèze G, Litvinova E, Septier F, Klatzmann D, Liblau R, Salomon BL. Continuous activation of autoreactive CD4+ CD25+ regulatory T cells in the steady state. J Exp Med. 2003 Sep 1;198(5):737-46. Epub 2003 Aug 25.

(7) Xu W et al, J Cancer Res Clin Oncol. 2013 Nov;139(11):1845-52

(8) Ladanyi A, Magy Onkol. 2013 Jun;57(2):85-95

(9) Zhang W et al, Gynecol Oncol. 2014 Jan 2. pii: S0090-8258(13)01427-3.

(10) Faghih Z et al, Immunol Lett. 2013 Dec 8;158(1-2):57-65.

(11) Aida K et al, Cancer Sci. 2013 Nov 30

(12) Huang XM et al, Cancer Sci. 2013 Nov

(13) Preston CC et al, PLoS One. 2013 Nov 14;8(11):e80063

(14) He M et al, Neuro Oncol. 2013 Jun; 15(6):727-34

(15) Muthu Raja KR et al, PLoS One. 2012;7(10):e47077

(16) Davidson S et al, Mod Pathol. 2013 Mar;26(3):448-55

(17) Huang Yet al, Digestion. 2012;86(4):329-37

(18) Memarian A, Tumour Biol. 2013 Feb;34(1):531-42

(19) Delhem et al, Expert Opin Biol Ther. 2010; 10(11) : 1563-1572

(20) Ouaguia et al. ISRN Hepatology. Volume 2013 (2013), Article ID 928485

(21) Carpentier et al, Am J Transplant. 2009; 9(9) : 2102-2112.

(22) Moralès et al, BRMI, Volume 2014 (2014), Article ID 290878

(23) Baumforth et al, Am J Pathol. 2008 Jul; 173(1):195-204

(24) Moralès et al, PlosOne, In press 2014

(25) Krausz LT et al, Ideggyogy Sz. 2013 Sep 30;66(9-10):343-8.

La présente description concerne également les éléments ci-après :

1. Anticorps dirigé contre la galectine 9 et inhibiteur de l'activité suppressive des lymphocytes T régulateurs.

2. Anticorps selon l'élément 1 qui se lie de manière spécifique à un épitope de séquence d'acides aminés SEQ ID NO : 9.

3. Anticorps selon l'un quelconque des éléments 1 ou 2 ayant la même zone de fixation que l'anticorps ayant pour CDRs les six CDRs définis par :

- la séquence d'acides aminés SEQ ID NO:2 dans la région H-CDR1,
- la séquence d'acides aminés SEQ ID NO:3 dans la région H-CDR2,
- la séquence d'acides aminés SEQ ID NO:4 dans la région H-CDR3,
- la séquence d'acides aminés SEQ ID NO:6 dans la région L-CDR1,
- la séquence d'acides aminés SEQ ID NO:7 dans la région L-CDR2,
- la séquence d'acides aminés SEQ ID NO:8 dans la région L-CDR3.

4. Anticorps selon l'un quelconque des éléments 1 ou 2 ayant pour CDRs les six CDRS définis par :

- la séquence d'acides aminés SEQ ID NO:2 dans la région H-CDR1,
- la séquence d'acides aminés SEQ ID NO:3 dans la région H-CDR2,
- la séquence d'acides aminés SEQ ID NO:4 dans la région H-CDR3,
- la séquence d'acides aminés SEQ ID NO:6 dans la région L-CDR1,
- la séquence d'acides aminés SEQ ID NO:7 dans la région L-CDR2,

- la séquence d'acides aminés SEQ ID NO:8 dans la région L-CDR3.

5. Anticorps selon les éléments 1 à 4, caractérisé en ce que la région variable de chaîne lourde dudit anticorps a la séquence d'acides aminés SEQ ID NO:1 et en ce que la région variable de chaîne légère dudit anticorps a la séquence d'acides aminés SEQ ID NO:5.

6. Anticorps selon l'élément 1 ayant la même zone de fixation que l'anticorps ayant pour CDRs les six CDRs définis par :

- la séquence d'acides aminés SEQ ID NO:11 dans la région H-CDR1,
- la séquence d'acides aminés SEQ ID NO:12 dans la région H-CDR2,
- la séquence d'acides aminés SEQ ID NO:13 dans la région H-CDR3,
- la séquence d'acides aminés SEQ ID NO:15 dans la région L-CDR1,
- la séquence d'acides aminés SEQ ID NO:16 dans la région L-CDR2,
- la séquence d'acides aminés SEQ ID NO:17 dans la région L-CDR3.

7. Anticorps selon l'élément 1 ayant pour CDRs les six CDRS définis par :

- la séquence d'acides aminés SEQ ID NO:11 dans la région H-CDR1,
- la séquence d'acides aminés SEQ ID NO:12 dans la région H-CDR2,
- la séquence d'acides aminés SEQ ID NO:13 dans la région H-CDR3,
- la séquence d'acides aminés SEQ ID NO:15 dans la région L-CDR1,
- la séquence d'acides aminés SEQ ID NO:16 dans la région L-CDR2,
- la séquence d'acides aminés SEQ ID NO:17 dans la région L-CDR3.

8. Anticorps selon l'un quelconque des éléments 1, 6 ou 7, caractérisé en ce que la région variable de chaîne lourde dudit anticorps a la séquence d'acides aminés SEQ ID NO:10 et en ce que la région variable de chaîne légère dudit anticorps a la séquence d'acides aminés SEQ ID NO:14.

9. Anticorps selon l'un quelconque des éléments 1 à 8 pour son utilisation dans le traitement de maladies associées à l'activité suppressive des lymphocytes T régulateurs.

10. Anticorps pour son utilisation selon l'élément 9, caractérisé en ce que ledit anticorps est utilisé dans le traitement de cancer.

11. Anticorps pour son utilisation selon l'élément 10, caractérisé en ce que le cancer est choisi dans le groupe constitué des Leucémies Myéloïdes Chroniques, cancer du colon, mélanome, cancer de l'utérus, cancer du sein, cancer du pancréas, cancers gastriques, cancer des ovaires, lymphome primaire du système nerveux central, myélomes multiples, cancer de la prostate, lymphome de Hodgkin et carcinome hépatocellulaire.

12. Anticorps pour son utilisation selon l'élément 10, caractérisé en ce que le cancer est un cancer viro-induit, de préférence choisi dans le groupe constitué des carcinomes du nasopharynx associés au Virus d'Epstein-Barr, des carcinomes hépatocellulaires liés au virus de l'hépatite C ou au virus de l'hépatite B.

13. Composition pharmaceutique, comprenant un anticorps selon l'un quelconque des éléments 1 à 8 et au moins un support pharmaceutiquement acceptable.

14. Produit de combinaison comprenant :

- un anticorps tel qu'il est défini dans l'un quelconque des éléments 1 à 8, et,
- un agent anticancéreux.

15. Produit de combinaison tel que défini à l'élément 14 pour une utilisation simultanée, séparée ou étalée dans le temps dans le traitement de cancer.

**Revendications**

1. Anticorps dirigé contre la galectine 9 et inhibiteur de l'activité suppressive des lymphocytes T régulateurs.

2. Anticorps selon la revendication 1 qui se lie de manière spécifique à un épitope de séquence d'acides aminés SEQ ID NO : 9.

3. Anticorps selon l'une quelconque des revendications 1 ou 2 ayant la même zone de fixation que l'anticorps ayant pour CDRs les six CDRs définis par :

- la séquence d'acides aminés SEQ ID NO:2 dans la région H-CDR1,
- la séquence d'acides aminés SEQ ID NO:3 dans la région H-CDR2,
- la séquence d'acides aminés SEQ ID NO:4 dans la région H-CDR3,
- la séquence d'acides aminés SEQ ID NO:6 dans la région L-CDR1,
- la séquence d'acides aminés SEQ ID NO:7 dans la région L-CDR2,
- la séquence d'acides aminés SEQ ID NO:8 dans la région L-CDR3.

4. Anticorps selon l'une quelconque des revendications 1 ou 2 ayant pour CDRs les six CDRS définis par :

- la séquence d'acides aminés SEQ ID NO:2 dans la région H-CDR1,
- la séquence d'acides aminés SEQ ID NO:3 dans la région H-CDR2,
- la séquence d'acides aminés SEQ ID NO:4 dans la région H-CDR3,
- la séquence d'acides aminés SEQ ID NO:6 dans la région L-CDR1,

- la séquence d'acides aminés SEQ ID NO:7 dans la région L-CDR2,
- la séquence d'acides aminés SEQ ID NO:8 dans la région L-CDR3.

5. Anticorps selon les revendications 1 à 4, **caractérisé en ce que** la région variable de chaîne lourde dudit anticorps a la séquence d'acides aminés SEQ ID NO:1 et **en ce que** la région variable de chaîne légère dudit anticorps a la séquence d'acides aminés SEQ ID NO:5.

6. Anticorps selon la revendication 1 ayant la même zone de fixation que l'anticorps ayant pour CDRs les six CDRs définis par :

- la séquence d'acides aminés SEQ ID NO:11 dans la région H-CDR1,
- la séquence d'acides aminés SEQ ID NO:12 dans la région H-CDR2,
- la séquence d'acides aminés SEQ ID NO:13 dans la région H-CDR3,
- la séquence d'acides aminés SEQ ID NO:15 dans la région L-CDR1,
- la séquence d'acides aminés SEQ ID NO:16 dans la région L-CDR2,
- la séquence d'acides aminés SEQ ID NO:17 dans la région L-CDR3.

7. Anticorps selon la revendication 1 ayant pour CDRs les six CDRS définis par :

- la séquence d'acides aminés SEQ ID NO:11 dans la région H-CDR1,
- la séquence d'acides aminés SEQ ID NO:12 dans la région H-CDR2,
- la séquence d'acides aminés SEQ ID NO:13 dans la région H-CDR3,
- la séquence d'acides aminés SEQ ID NO:15 dans la région L-CDR1,
- la séquence d'acides aminés SEQ ID NO:16 dans la région L-CDR2,
- la séquence d'acides aminés SEQ ID NO:17 dans la région L-CDR3.

8. Anticorps selon l'une quelconque des revendications 1, 6 ou 7, **caractérisé en ce que** la région variable de chaîne lourde dudit anticorps a la séquence d'acides aminés SEQ ID NO:10 et **en ce que** la région variable de chaîne légère dudit anticorps a la séquence d'acides aminés SEQ ID NO:14.

9. Anticorps selon l'une quelconque des revendications 1 à 8 pour son utilisation dans le traitement de maladies associées à l'activité suppressive des lymphocytes T régulateurs.

10. Composition pharmaceutique, comprenant un anticorps selon l'une quelconque des revendications 1 à 3, 5, 6 et 8 et au moins un support pharmaceutiquement acceptable.

11. Produit de combinaison comprenant :

- un anticorps tel qu'il est défini dans l'une quelconque des revendications 1 à 8, et,
- un agent anticancéreux.

12. Produit de combinaison tel que défini à la revendication 11 pour une utilisation simultanée, séparée ou étalée dans le temps dans le traitement de cancer.

Fig. 1

Fig. 2

Fig. 3

Fig. 4

**Fig. 5**

**Fig. 6**

Fig. 7

Fig. 8

**Fig. 9**

Fig. 10

Treg
Tconv (CD4+ CD25-)

Test statistique Rank Sum Mann Whitney
* p<=0.05, ** p<=0.001

**Fig. 11**

Test statistique Rank Sum Mann Whitney
* p<=0.05, ** p<=0.001

**Fig.12**

Test statistique Rank Sum Mann Whitney
* p<=0.05, ** p<=0.001

**Fig. 13**

Test statistique Rank Sum Mann Whitney
* p<=0.05, ** p<=0.001

Fig. 14

Test statistique Rank Sum Mann Whitney
* p<=0.05, ** p<=0.001

Fig. 15

Test statistique Rank Sum Mann Whitney
* p<=0.05, ** p<=0.001

**Fig. 16**

**Fig. 17**

**A**

**B**

Fig. 18

**A**

Volume Tumoral (mm3)

8000

6000

4000

2000

0

D14   D21   D23   D26   D28

Groupe 1: PBMC + Treg + IgG1
Groupe 2: PBMC + Treg + 1G3

**Jours**

**B**

volume Tumoral (mm³)

7000

6000

5000

4000

3000

2000

1000

0

D+5  D+7  D+9  D+12  D+14  D+16  D+19  D+21  D+23  D+26  D+28

IgG1

1G3

**Jours**

**Fig. 19**

C

D

**Fig. 19**

Test statistique Rank Sum Mann Whitney
* p<=0.05, ** p<=0.001

**Fig. 20**

**A**

**B**

**Fig. 21**

**Fig. 21**

Test statistique Rank Sum Mann Whitney
* p<=0.05, ** p<=0.001

**Fig. 22**

Test statistique Rank Sum Mann Whitney
* p<=0.05, ** p<=0.001

**Fig. 23**

Fig. 24

Fig. 25

**Fig. 26**

**Europäisches Patentamt**
**European Patent Office**
**Office européen des brevets**

# RAPPORT DE RECHERCHE EUROPEENNE

Numéro de la demande

EP 20 18 4087

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | CLÉMENT BARJON ET AL: "A novel monoclonal antibody for detection of galectin-9 in tissue sections: application to human tissues infected by oncogenic viruses", INFECTIOUS AGENTS AND CANCER, BIOMED CENTRAL LTD, LO, vol. 7, no. 1, 17 juillet 2012 (2012-07-17), page 16, XP021116920, ISSN: 1750-9378, DOI: 10.1186/1750-9378-7-16 * page 8/11, colonne de gauche - colonne de droite; figures 1-4 * | 1-3,6 | INV. C07K16/28 C07K16/30 A61K39/395 A61P35/00 |
| A | WO 2010/084999 A1 (PROTEGENE INC [JP]; UNIV OKAYAMA NAT UNIV CORP [JP]; JUNTENDO EDUCATIO) 29 juillet 2010 (2010-07-29) * revendications 1-11; figures 8,9; exemples 2,5-7 * | 1-12 | |
| X | JIHÈ NE KLIBI ET AL: "Blood diffusion and Th1-suppressive effects of galectin-9-containing exosomes released by Epstein-Barr virus-infected nasopharyngeal carcinoma cells", BLOOD, vol. 113, no. 9, 26 février 2009 (2009-02-26), pages 1957-1966, XP55538746, DOI: 10.1182/blood-2008-02- | 9-12 | DOMAINES TECHNIQUES RECHERCHES (IPC) C07K A61K |
| A | * le document en entier * | 1-8 | |

-/--

Le présent rapport a été établi pour toutes les revendications

| Lieu de la recherche | Date d'achèvement de la recherche | Examinateur |
|---|---|---|
| La Haye | 28 août 2020 | Le Flao, Katell |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 2

Europäisches
Patentamt

European
Patent Office

Office européen
des brevets

# RAPPORT DE RECHERCHE EUROPEENNE

Numéro de la demande

EP 20 18 4087

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | KADOWAKI TAKESHI ET AL: "Galectin-9 signaling prolongs survival in murine lung-cancer by inducing macrophages to differentiate into plasmacytoid dendritic cell-like macrophages", CLINICAL IMMUNOLOGY, vol. 142, no. 3, 2012, pages 296-307, XP028898525, ISSN: 1521-6616, DOI: 10.1016/J.CLIM.2011.11.006 * page 297, colonne de gauche; figure 1 * * page 298, colonne de droite * ----- | 1-12 | |
| A | WO 2012/177788 A1 (JOLLA INST ALLERGY IMMUNOLOG [US]; CROFT MICHAEL [US]; MADIREDDI SHRAV) 27 décembre 2012 (2012-12-27) * exemples 1-7 * ----- | 1-12 | |
| T | CLAIRE LHUILLIER ET AL: "Characterization of neutralizing antibodies reacting with the 213-224 amino-acid segment of human galectin-9", PLOS ONE, vol. 13, no. 9, 11 septembre 2018 (2018-09-11), page e0202512, XP055538758, DOI: 10.1371/journal.pone.0202512 * le document en entier * ----- | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| La Haye | 28 août 2020 | Le Flao, Katell |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE
## RELATIF A LA DEMANDE DE BREVET EUROPEEN NO.

EP 20 18 4087

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-08-2020

| Document brevet cité au rapport de recherche | | Date de publication | Membre(s) de la famille de brevet(s) | | Date de publication |
|---|---|---|---|---|---|
| WO 2010084999 | A1 | 29-07-2010 | JP | 5569946 B2 | 13-08-2014 |
| | | | JP | 2012515766 A | 12-07-2012 |
| | | | WO | 2010084999 A1 | 29-07-2010 |
| WO 2012177788 | A1 | 27-12-2012 | US | 2014234320 A1 | 21-08-2014 |
| | | | WO | 2012177788 A1 | 27-12-2012 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 1586325 A
- WO 9514785 A
- WO 9622378 A

- US 5882877 A
- WO 9845322 A
- EP 0173494 A

**Littérature non-brevet citée dans la description**

- **SEKI et al.** Galectin-9 suppresses the generation of Th17, promotes the induction of regulatory T cells, and regulates experimental autoimmune arthritis. *Clin Immunol,* Avril 2008, vol. 127 (1), 78-88
- **GROSSMAN WJ ; VERBSKY JW ; BARCHET W ; COLONNA M ; ATKINSON JP ; LEY TJ.** Human T regulatory cells can use the perforin pathway to cause autologous target cell death. *Immunity,* Octobre 2004, vol. 21 (4), 589-601
- **GARIN MI ; CHU CC ; GOLSHAYAN D ; CERNU-DA-MOROLLÓN E ; WAIT R ; LECHLER RI.** Galectin-1: a key effector of regulation mediated by CD4+CD25+ T cells. *Blood.,* 01 Mars 2007, vol. 109 (5), 2058-65
- **JOHNSON BD ; JING W ; ORENTAS RJ.** CD25+ regulatory T cell inhibition enhances vaccine-induced immunity to neuroblastoma. *J Immunother.,* Février 2007, vol. 30 (2), 203-14
- **MCHUGH RS ; WHITTERS MJ ; PICCIRILLO CA ; YOUNG DA ; SHEVACH EM ; COLLINS M ; BYRNE MC.** CD4(+)CD25(+) immunoregulatory T cells: gene expression analysis reveals a functional role for the glucocorticoid-induced TNF receptor. *Immunity,* Février 2002, vol. 16 (2), 311-23
- **WING K ; ONISHI Y ; PRIETO-MARTIN P ; YAMAGUCHI T ; MIYARA M ; FEHERVARI Z ; NOMURA T ; SAKAGUCHI S.** CTLA-4 control over Foxp3+ regulatory T cell function. *Science,* 10 Octobre 2008, vol. 322 (5899), 271-5
- **ZAHRAN AM et al.** *Int J Clin Oncol.,* 26 Septembre 2013
- **FISSON S1 ; DARRASSE-JÈZE G ; LITVINOVA ; SEPTIER F ; KLATZMANN D ; LIBLAU R ; SALO-MON BL.** Continuous activation of autoreactive CD4+ CD25+ regulatory T cells in the steady state. *J Exp Med.,* 01 Septembre 2003, vol. 198 (5), 737-46

- **XU W et al.** *J Cancer Res Clin Oncol,* Novembre 2013, vol. 139 (11), 1845-52
- **LADANYI A.** *Magy Onkol.,* Juin 2013, vol. 57 (2), 85-95
- **ZHANG W et al.** *Gynecol Oncol.,* 02 Janvier 2014, 01427-3
- **FAGHIH Z et al.** *Immunol Lett.,* 08 Décembre 2013, vol. 158 (1-2), 57-65
- **AIDA K et al.** *Cancer Sci.,* 30 Novembre 2013
- **HUANG XM et al.** *Cancer Sci.,* Novembre 2013
- **PRESTON CC et al.** *PLoS One.,* 14 Novembre 2013, vol. 8 (11), e80063
- **HE M et al.** *Neuro Oncol.,* Juin 2013, vol. 15 (6), 727-34
- **MUTHU RAJA KR et al.** *PLoS One.,* 2012, vol. 7 (10), e47077
- **DAVIDSON S et al.** *Mod Pathol.,* Mars 2013, vol. 26 (3), 448-55
- **HUANG YET.** *Digestion,* 2012, vol. 86 (4), 329-37
- **MEMARIAN A.** *Tumour Biol.,* Février 2013, vol. 34 (1), 531-42
- **DELHEM et al.** *Expert Opin Biol Ther.,* 2010, vol. 10 (11), 1563-1572
- **OUAGUIA et al.** *ISRN Hepatology,* 2013, vol. 2013
- **CARPENTIER et al.** *Am J Transplant,* 2009, vol. 9 (9), 2102-2112
- **MORALÈS et al.** *BRMI,* 2014, vol. 2014
- **BAUMFORTH et al.** *Am J Pathol,* Juillet 2008, vol. 173 (1), 195-204
- **MORALÈS et al.** *PlosOne,* 2014
- **KRAUSZ LT et al.** *Ideggyogy Sz.,* 30 Septembre 2013, vol. 66 (9-10), 343-8

SEQUENCE LISTING

<110> Université de Lille
Université Paris-Saclay
Centre National de la Recherche Scientifique
Institut Gustave Roussy
Société Cellvax Pharma

<120> ANTIBODY WHICH IS DIRECTED AGAINST GALECTIN-9 AND IS AN INHIBITOR
OF THE SUPPRESSOR ACTIVITY OF REGULATORY T LYMPHOCYTES

<130> GAL 9 DIV

<160> 17

<170> PatentIn version 3.5

<210> 1
<211> 148
<212> PRT
<213> Artificial Sequence

<220>
<223> fragment of antibody

<400> 1

Met Lys Cys Ser Trp Gly Ile Phe Phe Leu Leu Ser Val Thr Ala Gly
1               5                   10                  15


Val His Ser Lys Val Gln Leu Gln Gln Ser Gly Ala Glu Leu Val Lys
            20                  25                  30


Pro Gly Ala Ser Val Lys Leu Ser Cys Lys Ala Ser Gly Tyr Thr Phe
            35                  40                  45


Thr Asp Tyr Thr Ile His Trp Val Lys Gln Arg Ser Gly Gln Gly Leu
            50                  55                  60


Glu Trp Ile Gly Trp Phe Tyr Pro Gly Ser His Ser Ile Lys Tyr Asn
65                  70                  75                  80


Glu Gln Phe Lys Asp Arg Ala Thr Leu Thr Ala Asp Lys Ser Ser Ser
                85                  90                  95


Thr Val Tyr Met Glu Leu Ser Arg Leu Thr Ser Glu Asp Ser Ala Val
                100                 105                 110


Tyr Phe Cys Thr Arg His Gly Gly Tyr Asp Gly Phe Asp Tyr Trp Gly
            115                 120                 125


Gln Gly Thr Thr Leu Thr Val Ser Ser Ala Lys Thr Thr Pro Pro Ser
            130                 135                 140

Val Tyr Pro Leu
145


<210> 2
<211> 10
<212> PRT
<213> Artificial Sequence

<220>
<223> fragment of antibody

<400> 2

Gly Tyr Thr Phe Thr Asp Tyr Thr Ile His
1               5               10


<210> 3
<211> 18
<212> PRT
<213> Artificial Sequence

<220>
<223> fragment of antibody

<400> 3

Trp Phe Tyr Pro Gly Ser His Ser Ile Lys Tyr Asn Glu Gln Phe Lys
1               5               10              15

Asp Arg


<210> 4
<211> 9
<212> PRT
<213> Artificial Sequence

<220>
<223> fragment of antibody

<400> 4

His Gly Gly Tyr Asp Gly Phe Asp Tyr
1               5


<210> 5
<211> 153
<212> PRT
<213> Artificial Sequence

<220>
<223> fragment of antibody

<400> 5

Leu Asp Gly Gly Lys Met Asp Ser Gln Ala Gln Val Leu Met Leu Leu
1               5               10              15

Leu Leu Trp Val Ser Gly Thr Cys Gly Asp Ile Val Met Ser Gln Ser
            20                  25              30

Pro Ser Ser Leu Ala Val Ser Val Gly Glu Lys Ile Thr Met Ser Cys
            35                  40              45

Lys Ser Ser Gln Ser Leu Phe Tyr Ser Thr Asn Gln Lys Asn Tyr Leu
            50              55              60

Ala Trp Tyr Gln Gln Lys Pro Gly Gln Ser Pro Lys Leu Leu Ile Tyr
65              70              75                  80

Trp Ala Ser Thr Arg Glu Ser Gly Val Pro Asp Arg Phe Thr Gly Ser
                85              90              95

Gly Ser Gly Thr Asp Phe Thr Leu Thr Ile Ser Ser Val Lys Ala Glu
            100             105             110

Asp Leu Ala Val Tyr Tyr Cys Gln Gln Tyr Tyr Tyr Phe Pro Tyr Thr
            115             120             125

Phe Gly Gly Gly Thr Lys Leu Glu Ile Lys Arg Ala Asp Ala Ala Pro
    130             135             140

Thr Val Ser Ile Phe Pro Pro Ser Ser
145             150

<210>  6
<211>  17
<212>  PRT
<213>  Artificial Sequence

<220>
<223>  fragment of antibody

<400>  6

Lys Ser Ser Gln Ser Leu Phe Tyr Ser Thr Asn Gln Lys Asn Tyr Leu
1               5               10              15

Ala

<210>  7
<211>  7
<212>  PRT
<213>  Artificial Sequence

<220>
<223>  fragment of antibody

```
<400>   7

Trp Ala Ser Thr Arg Glu Ser
1               5


<210>   8
<211>   9
<212>   PRT
<213>   Artificial Sequence

<220>
<223>   antibody

<400>   8

Gln Gln Tyr Tyr Tyr Phe Pro Tyr Thr
1               5


<210>   9
<211>   13
<212>   PRT
<213>   Artificial Sequence

<220>
<223>   fragment of galectin 9

<400>   9

Thr Pro Ala Ile Pro Pro Met Met Tyr Pro His Pro Ala
1               5               10


<210>   10
<211>   175
<212>   PRT
<213>   Artificial Sequence

<220>
<223>   VH 2E12

<400>   10

Met Gly Trp Ser Phe Ile Ile Leu Leu Ser Val Thr Ala Gly Val His
1               5               10              15


Ser Lys Val Gln Leu Gln Gln Ser Gly Ala Glu Leu Val Lys Pro Gly
            20              25              30


Ala Ser Val Lys Leu Ser Cys Lys Ala Ser Gly Tyr Thr Phe Thr Glu
            35              40              45


Tyr Thr Ile His Trp Val Lys Gln Arg Ser Gly Gln Gly Leu Glu Trp
            50              55              60


Ile Gly Trp Phe Tyr Pro Gly Ser Gly Ser Met Glu Tyr Asn Glu Lys
65              70              75              80
```

```
Phe Asp Lys Ala Thr Leu Thr Ala Asp Asn Ser Ser Ser Thr Val Tyr
                85                    90              95

Met Glu Leu Ser Arg Leu Thr Ser Glu Asp Ser Ala Val Tyr Phe Cys
                100                   105             110

Glu Arg His Gly Gly Tyr Asp Gly Phe Asp Tyr Trp Gly Gln Gly Thr
                115                   120             125

Thr Leu Thr Val Ser Ser Ala Lys Thr Thr Pro Pro Ser Val Tyr Pro
                130                   135             140

Leu Ile Phe Leu Glu Asp Leu Leu Gln Tyr Ser Gln Leu Pro Trp Lys
145                   150                   155             160

Ile Asp Val Leu Leu Leu Phe Ser Gln Asp Phe Gln Ala Val Tyr
                165                   170             175


<210>  11
<211>  10
<212>  PRT
<213>  Artificial Sequence

<220>
<223>  H-CDR1 2E12

<400>  11

Gly Tyr Thr Phe Thr Glu Tyr Thr Ile His
1               5               10


<210>  12
<211>  16
<212>  PRT
<213>  Artificial Sequence

<220>
<223>  H-CDR2 2E12

<400>  12

Trp Phe Tyr Pro Gly Ser Gly Ser Met Glu Tyr Asn Glu Lys Phe Asp
1               5               10              15


<210>  13
<211>  9
<212>  PRT
<213>  Artificial Sequence

<220>
<223>  H-CDR3 2E12

<400>  13
```

His Gly Gly Tyr Asp Gly Phe Asp Tyr
1               5


<210>   14
<211>   153
<212>   PRT
<213>   Artificial Sequence

<220>
<223>   VL 2E12

<400>   14

Leu Asp Gly Gly Lys Met Asp Ser Gln Ala Gln Val Leu Met Leu Leu
1               5                   10                  15


Leu Leu Trp Val Ser Gly Thr Cys Gly Asp Ile Val Met Ser Gln Ser
            20                  25                  30


Pro Ser Ser Leu Ala Val Ser Val Gly Glu Lys Val Thr Met Ser Cys
            35                  40                  45


Lys Ser Ser Gln Ser Leu Leu Tyr Ser Asn Asn Gln Lys Asn Tyr Leu
            50                  55                  60


Ala Trp Tyr Gln Gln Lys Pro Gly Gln Ser Pro Lys Leu Leu Ile Tyr
65                  70                  75                  80


Trp Ala Ser Thr Arg Gly Ser Gly Val Pro Asp Arg Phe Thr Gly Ser
                85                  90                  95


Gly Ser Gly Thr Asp Phe Thr Leu Thr Ile Ser Ser Val Lys Ala Glu
                100                 105                 110


Asp Leu Ala Ile Tyr Tyr Cys Gln Gln Tyr Tyr Ser Tyr Pro Phe Thr
                115                 120                 125


Phe Gly Gly Gly Thr Lys Leu Glu Ile Lys Arg Ala Asp Ala Ala Pro
                130                 135                 140


Thr Val Ser Ile Phe Pro Pro Ser Ser
145                 150


<210>   15
<211>   17
<212>   PRT
<213>   Artificial Sequence

<220>
<223>   L-CDR1 2E12

<400>   15

34

```
Lys Ser Ser Gln Ser Leu Leu Tyr Ser Asn Asn Gln Lys Asn Tyr Leu
1               5                   10                  15

Ala


<210>  16
<211>  7
<212>  PRT
<213>  Artificial Sequence

<220>
<223>  LCDR2 2E12

<400>  16

Trp Ala Ser Thr Arg Gly Ser
1               5


<210>  17
<211>  9
<212>  PRT
<213>  Artificial Sequence

<220>
<223>  L-CDR3 2E12

<400>  17

Gln Gln Tyr Tyr Ser Tyr Pro Phe Thr
1               5
```